# EUROPEAN PATENT SPECIFICATION

(11) **EP 3 965 730 B1**
(45) Date of publication and mention of the grant of the patent: **08.07.2026**
(21) Application number: 19927892.0
(22) Date of filing: 09.05.2019
(51) Int. Cl.: A61K 9/00, A61N 1/362, C09D 175/02, C08G 71/02, A61N 1/375, A61N 1/39

(54) **ANTIBIOTIC ELUTING POLY (ESTER UREA) FILMS FOR INFECTION CONTROL OF IMPLANTABLE MEDICAL DEVICES**
ANTIBIOTISCHE ELUIERENDE POLY(ESTERHARNSTOFF)FILME ZUR INFEKTIONSKONTROLLE IMPLANTIERBARER MEDIZINPRODUKTE
FILMS DE POLY(ESTER-URÉE) D'ÉLUTION D'ANTIBIOTIQUE POUR LA LUTTE CONTRE L'INFECTION DE DISPOSITIFS MÉDICAUX IMPLANTABLES

(43) Date of publication of application: 16.03.2022
(73) Proprietor: The University of Akron, Akron, OH 44325 (US); 21st Century Medical Technologies, LLC, Burlington, NC 27215 (US)
(72) Inventor: BECKER, Matthew, Chapel Hill, NC 27517 (US); NIKAM, Shantanu Pratap, Akron, Ohio 44304 (US); ALFARO, Arthur, Holly Springs, North Carolina 27540 (US)
(74) Representative: Schütte, Gearoid
(86) International application number: PCT/US2019/031405
(87) International publication number: WO 2020/226646

(56) References cited:
- EP-A2- 0 882 461
- WO-A1-2019/032541
- WO-A2-2007/146119
- WO-A2-2010/088682
- US-A1- 2004 236 416
- US-A1- 2005 147 690
- US-A1- 2007 128 250
- US-A1- 2007 129 792
- US-B2- 9 415 225
- US-B2- 9 745 414
- A. BERNI, M. MENNIG, H. SCHMIDT: "Doctor Blade", SOL-GEL TECHNOLOGIES FOR GLASS PRODUCERS AND USERS, 2004, New York, pages 89 - 90, XP055760012, Retrieved from the Internet <URL:https://link.springer.com/chapter/10.1007/978-0-387-88953-5_10> [retrieved on 20190621]

## Description

### FIELD OF THE INVENTION

The present invention relates to a drug loaded degradable amino acid based poly(ester urea) film for drug delivery.

### BACKGROUND OF THE INVENTION

Poly (ester ureas) for shape memory and drug delivery and methods for their synthesis and use are disclosed in WO 2019/032541A1.

Implant-associated infections acquired during implantation procedures are a major complication that results in chronic and fatal infections. Despite timely application of infection control practices, and administration of antibiotic prophylaxis during implantation, the occurrence of these infections has not declined and is still unacceptably high. The cardiac implantable electronic device (CIED) infection is one such type and is associated with significant morbidity and poor functional outcomes. A cardiac implantable electronic device (CIED) includes pacemakers, cardiac resynchronization therapy (CRT) devices and implantable cardioverter defibrillators (ICDs). These devices are used in patients suffering from the irregular or arrhythmic heartbeat. About 400,000 patients in the U.S. undergo cardiac implant procedures every year. With pacemaker infection risk is 0.5-1% with ICDs is estimated to be 1.7% within first 6 months. The risk is highest in CRT-recipients, 9.5% over 2 years.

The microorganisms that cause CIED infections may be acquired either endogenously from the skin of patients or exogenously from the hospital environment. Early diagnosis of these infections is difficult. Cell cultures from CIED-pocket tissue taken at the time of CIED removal indicate that Staphylococcus species are responsible for ~70% of CIED infections. Formation of biofilm on the implant renders it useless and hence replacement is the only option. Extraction of an infected CIED is a highly invasive procedure that requires a substantial amount of resources and antibiotic treatment. Treatment of these infections typically cost the facility an average of $52,000 but may exceed well over $100,000 and puts an annual healthcare burden of ~ 1 billion dollars on the US.

The conventional, systemic antibiotic delivery is less efficacious in many cases as nearly 1 million implant-associated infections occur every year. Localized antibiotic delivery must be employed in a supplement to systemic administration. The advantages of using locally delivered antibiotics include the delivery of a high concentration of antibiotics to a localized area without surpassing systemic toxicity, prolonged release over time, minimal risk of developing bacterial resistance, reduction of gastrointestinal and other adverse effects related to systemic antibiotics as well as reduced risk of patient noncompliance with antibiotic regimens. Locally released antibiotic can eliminate bacteria in their dormant stages preventing severe chronic infection and the need for CIED replacement.

What is needed in the art is a polymer envelope for a CIED or other device implanted in the body of a patient that can locally deliver antibiotics that could prove effective in reducing instances of infection with the implanted device and/or locally deliver one or more other drugs to improve the effectiveness of the implanted device.

### SUMMARY OF THE INVENTION

The invention is set out in the appended set of claims. In one or more embodiments, the present invention provides a polymer envelope for a CIED or other device implanted in the body of a patient, which can locally deliver antibiotics that could prove effective in reducing instances of infection with the implanted device and/or locally deliver one or more other drugs to improve the effectiveness of the implanted device. In some embodiments, the invention is directed to a drug-loaded amino acid based poly(ester urea) film that produces localized drug delivery for implanted devices. In some embodiments, the present invention is directed to an antibiotic-loaded L-valine poly(ester urea) film for localized antibiotic delivery for
CIEDs or other devices during implantation. In some other embodiments, the present invention is directed to an implantable medical device comprising a drug-loaded degradable amino acid based poly(ester urea) film to improve the effectiveness of the of the implanted device. In some of these embodiments, the present invention is directed to an implantable medical device comprising an antibiotic-loaded degradable L-valine based poly(ester urea) film for localized antibiotic delivery during implantation to reduce instances of infection with the implanted device. In still other embodiments, the present invention related to a method of making the drug-loaded amino acid based poly(ester urea) films described above.

In a first aspect, the present invention is directed to a drug loaded degradable amino acid based poly(ester urea) (PEU) film for drug delivery comprising an amino acid based poly(ester urea) polymer and a drug. In one or more of these embodiments, the amino acid based poly(ester urea) polymer comprises residues of two or more amino acid based diester monomer segments joined by urea linkages. In some of these embodiments, the two or more amino acid based diester monomer segments comprise the residues of two amino acids separated by from 1 to 20 carbon atoms.

In one or more embodiments, the drug loaded degradable amino acid based PEU film of the present invention includes any one or more of the above referenced embodiments of the first aspect of the present invention wherein the two or more amino acid based diester monomer segments comprise the reaction product of two amino acids and a C1-C20 diol. In one or more embodiments, the drug loaded degradable amino acid based PEU film of the present invention includes any one or more of the above referenced embodiments of the first aspect of the present invention wherein the two amino acids are selected from the group consisting of alanine (ala - A ); arginine (arg - R); asparagine (asn - N); aspartic acid (asp - D); cysteine (cys - C); glutamine (gln - Q); glutamic acid (glu - E); glycine (gly - G); histidine (his - H); isoleucine (ile - I); leucine (leu - L); lysine (lys - K); methionine (met - M); phenylalanine (phe - F); serine (ser - S); threonine (thr - T); tryptophan (trp - W); tyrosine (tyr - Y); valine (val - V) and combinations thereof. In one or more embodiments, the drug loaded degradable amino acid based PEU film of the present invention includes any one or more of the above
referenced embodiments of the first aspect of the present invention wherein the two amino acids are both L-valine. In one or more embodiments, the drug loaded degradable amino acid based PEU film of the present invention includes any one or more of the above referenced embodiments of the first aspect of the present invention wherein the C1-C20 diol is selected from 1,6-hexanediol, 1,8-octanediol, 1,9-nonanediol, 1,10-decanediol, 1,11-undecanediol, 1,12-dodecanediol, 1,13-tridecanediol, 1,14-tetradecanediol, 1,15-pentadecanediol, 1,16-hexadecanediol, 1,17-heptadecanediol, 1,18-octadecanediol, 1,19-nonadecanediol, 1,20-icosanediol, 2-butene-1,4-diol, 3,4-dihydroxy-1-butene, 7-octene-1,2-diol, 3-hexene-1,6-diol, 1,4-butynediol, trimethylolpropane allyl ether, 3-allyloxy-1,2-propanediol, 2,4-hexadiyne-1,6-diol, 2-hydroxymethyl-1,3-propanediol, and combinations thereof.

In one or more embodiments, the drug loaded degradable amino acid based PEU film of the present invention includes any one or more of the above referenced embodiments of the first aspect of the present invention wherein the two or more amino acid based diester monomer segments have the formula: 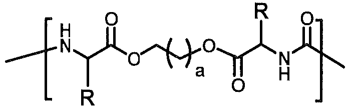
wherein R is -CH₃, -(CH₂)₃NHC(NH₂)C=NH, -CH₂CONH₂,-CH₂COOH, -CH₂SH, -(CH₂)₂COOH, -(CH₂)₂CONH₂, -NH₂, -CH₂C=CH-N=CH-NH, -CH(CH₃)CH₂CH₃, -CH₂CH(CH₃)₂, -(CH₂)₄NH₂, -(CH₂)₂SCH₃, -CH₂Ph, -CH₂OH, -CH(OH)CH₃, -CH₂-C=CH-NH-Ph, -CH₂-Ph-OH,-CH(CH₃)₂, or -CH₂C₆H₄OCH₂C₆H₅ and a is an integer from 1 to 20. In one or more embodiments, the drug loaded degradable amino acid based PEU film of the present invention includes any one or more of the above referenced embodiments of the first aspect of the present invention wherein the two or more amino acid based diester monomer segments comprise the residues of two L-valine molecules separated by from 1 to 20 carbon atoms. In one or more embodiments, the drug loaded degradable amino acid based PEU film of the present invention includes any one or more of the above referenced embodiments of the first aspect of the present invention wherein the degradable amino acid based poly(ester urea) polymer has the formula: 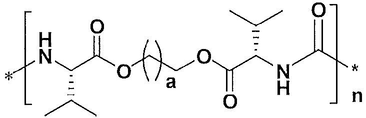
where a is an integer from 1 to 20 and n is an integer from 20 to 300.

In one or more embodiments, the drug loaded degradable amino acid based PEU film of the present invention includes any one or more of the above referenced embodiments of the first aspect of the present invention wherein the drug is selected from the group consisting of antibiotics, cancer drugs, antipsychotics, antidepressants, sleep aids, tranquillizers, anti-Parkinson's drugs, mood stabilizers, pain killers, anti-inflammatories, antimicrobials, and combinations thereof. In one or more embodiments, the drug loaded degradable amino acid based PEU film of the present invention includes any one or more of the above referenced embodiments of the first aspect of the present invention wherein the drug is an antibiotic selected from the group consisting of lipopeptides, fluoroquinolone, lipoglycopeptides, cephalosporins, penicillins, monobactams, carbapenems, macrolide antibiotics, lincosamides, streptogramins, aminoglycoside antibiotics, quinolone antibiotics, sulfonamides, tetracycline antibiotics, chloraphenicol, metronidazole, tinidazole, nitrofurantoin, glycopeptides, oxazolidinones, rifamycins, polypeptides, tuberactinomycins, and combinations or pharmaceutically acceptable salts thereof. In one or more embodiments, the drug is cefazolin sodium or a pharmaceutically acceptable salt thereof.

In one or more embodiments, the drug loaded degradable amino acid based PEU film of the present invention includes any one or more of the above referenced embodiments of the first aspect of the present invention comprising from 1 weight percent to 50 weight percent, preferably from 1 weight percent to 10 weight percent, and more preferably from 1 weight percent to 5 weight percent of the drug. In one or more embodiments, the drug loaded degradable amino acid based PEU film of the present invention includes any one or more of the above referenced embodiments of the first aspect of the present invention comprising from 2 weight percent to 10 weight percent of the drug. In one or more embodiments, the drug loaded degradable amino acid based PEU film of the present invention includes any one or more of the above referenced embodiments of the first aspect of the present invention wherein the drug is substantially uniformly distributed throughout the degradable amino acid based poly(ester urea) polymer.

In one or more embodiments, the drug loaded degradable amino acid based PEU film of the present invention includes any one or more of the above referenced embodiments of the first aspect of the present invention having a thickness of from 1µm to 1000 µm, preferably from 10 µm to 500 µm, and more preferably from 10 µm to 150 µm. In one or more embodiments, the drug loaded degradable amino acid based PEU film of the present invention includes any one or more of the above referenced embodiments of the first aspect of the present invention having a thickness of from 40 µm to 140 µm.

In a second aspect, the present invention is directed to an implantable medical device comprising the degradable amino acid based poly(ester urea) film described above. In some embodiments, the implantable medical device is may include, without limitation, cardiac implantable electronic devices (CIEDs), pace makers, implantable cardioverter defibrillators (ICDs), artificial heart valves, surgical mesh, hernia mesh films, transvaginal mesh, transabdominal mesh, sutures, shunts, stents, adhesion barriers, tissue scaffolds, auditory implants, ear tubes, cochlear implants, neurological implants, neural implants, implantable gastric stimulators, diaphragmatic/phrenic nerve stimulators, orthopedic implants, joint replacement appliances, spinal fusion hardware, surgical hardware for fracture repair (metal plates, pins, screws, rods, etc.), contraceptive implants, intra-uterine devices (IUDs), or cosmetic implants, implantable prostheses, and combinations thereof. In some embodiments, the implantable medical device comprises a cardiac implantable electronic device (CIED). In one or more embodiments, the degradable amino acid based poly(ester urea) film described above is applied implantable medical device by blade coating, lamination, dip coating, solvent coating, spray coating, extrusion, injection molding, or a combination thereof. In some embodiments, the implantable medical device is wrapped in or encased in the degradable amino acid based poly(ester urea) film described above.

In a third aspect, the present invention is directed to a method of making the degradable amino acid based poly(ester urea) film for drug delivery described above comprising: preparing a suitable substrate; preparing a degradable amino acid based poly(ester urea) polymer; selecting a drug to be delivered and dissolving it in a co-solvent for the drug and the degradable amino acid based poly(ester urea) polymer; dissolving the degradable amino acid based poly(ester urea) polymer in the drug solution; and forming the amino acid based poly(ester urea) polymer and drug solution into a film on the substrate. In some embodiments, the method further comprising allowing the film to dry and separating it from the substrate.

In one or more embodiments, the method of the present invention includes any one or more of the above referenced embodiments of the third aspect of the present invention wherein the substrate comprises poly(ethylene terephthalate) (PET), polytetrafluoroethene (PTFE) (TEFLON^{™}), poly(4,4'-oxydiphenylene-pyromellitimide) (KAPTON^{™}), polypropylene, or polyethylene. In one or more of these embodiments, the substrate comprises an implantable medical device.

In one or more embodiments, the method of the present invention includes any one or more of the above referenced embodiments of the third aspect of the present invention wherein the step of preparing a degradable amino acid based poly(ester urea) polymer comprises: reacting a C1-C20 diol with an two amino acids in the presence of an acid to form a counter ion protected amino acid based diester monomer; and reacting the counter ion protected amino acid based diester monomer with a urea bond forming compound to produce the degradable amino acid based poly(ester urea) polymer. In one or more embodiments, the method of the present invention includes any one or more of the above referenced embodiments of the third aspect of the present invention wherein the acid is selected from p-toluenesulfonic acid, HCl, and combinations thereof.

In one or more embodiments, the method of the present invention includes any one or more of the above referenced embodiments of the third aspect of the present invention wherein each of the two amino acids are selected from the group consisting of alanine (ala - A ); arginine (arg - R); asparagine (asn - N); aspartic acid (asp - D); cysteine (cys - C); glutamine (gln - Q); glutamic acid (glu - E); glycine (gly - G); histidine (his - H); isoleucine (ile - I); leucine (leu - L); lysine (lys - K); methionine (met - M); phenylalanine (phe - F); serine (ser - S); threonine (thr - T); tryptophan (trp - W); tyrosine (tyr - Y); valine (val - V) and combinations thereof. In one or more embodiments, the method of the present invention includes any one or more of the above referenced embodiments of the third aspect of the present invention wherein the two amino acids are both L-valine. In one or more embodiments, the method of the present invention includes any one or more of the above referenced embodiments of the third aspect of the present invention wherein the C1-C20 diol is selected from 1,6-hexanediol, 1,8-octanediol, 1,9-nonanediol, 1,10-decanediol, 1,11-undecanediol, 1,12-dodecanediol, 1,13-tridecanediol, 1,14-tetradecanediol, 1,15-pentadecanediol, 1,16-hexadecanediol, 1,17-heptadecanediol, 1,18-octadecanediol, 1,19-nonadecanediol, 1,20-icosanediol, 2-butene-1,4-diol, 3,4-dihydroxy-1-butene, 7-octene-1,2-diol, 3-hexene-1,6-diol, 1,4-butynediol, trimethylolpropane allyl ether, 3-allyloxy-1,2-propanediol, 2,4-hexadiyne-1,6-diol, 2-hydroxymethyl-1,3-propanediol, and combinations thereof.

In one or more embodiments, the method of the present invention includes any one or more of the above referenced embodiments of the third aspect of the present invention wherein the counter ion protected amino acid based diester monomer has the formula: 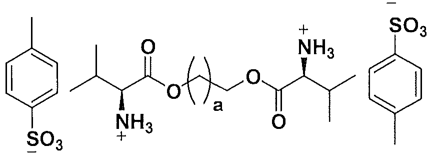 where a is an integer from 1 to 20.

In one or more embodiments, the method of the present invention includes any one or more of the above referenced embodiments of the third aspect of the present invention wherein the urea bond forming compound is phosgene, diphosgene, or triphosgene.

In one or more embodiments, the method of the present invention includes any one or more of the above referenced embodiments of the third aspect of the present invention wherein the drug to be delivered is selected from the group consisting of antibiotics, cancer drugs, antipsychotics, antidepressants, sleep aids, tranquillizers, anti-Parkinson's drugs, mood stabilizers, pain killers, anti-inflammatories, antimicrobials, and combinations thereof. In one or more embodiments, the method of the present invention includes any one or more of the above referenced embodiments of the third aspect of the present invention wherein the drug to be delivered is an antibiotic selected from the group consisting of lipopeptides, fluoroquinolone, lipoglycopeptides, cephalosporins, penicillins, monobactams, carbapenems, macrolide antibiotics, lincosamides, streptogramins, aminoglycoside antibiotics, quinolone antibiotics, sulfonamides, tetracycline antibiotics, chloraphenicol, metronidazole, tinidazole, nitrofurantoin, glycopeptides, oxazolidinones, rifamycins, polypeptides, tuberactinomycins, and combinations or pharmaceutical salts thereof. In one or more embodiments, the method of the present invention includes any one or more of the above referenced embodiments of the third aspect of the present invention wherein the drug to be delivered is selected from the group consisting of cefazolin sodium and pharmaceutically acceptable salts thereof.

In one or more embodiments, the method of the present invention includes any one or more of the above referenced embodiments of the third aspect of the present invention wherein the co-solvent for the drug and the degradable amino acid based poly(ester urea) polymer is selected from the group consisting of polar solvents, methanol, ethanol, isopropanol, acetone dioxane, THF, and combinations thereof. In one or more of these embodiments, the co-solvent for the drug and the degradable amino acid based poly(ester urea) polymer is methanol.

In one or more embodiments, the method of the present invention includes any one or more of the above referenced embodiments of the third aspect of the present invention wherein the ratio of the degradable amino acid based poly(ester urea) polymer to the drug in the solution is from 100:1 to 1:1, preferably from 10:1 to 11; and more preferably from 5:1 to 1:1 by weight.

In one or more embodiments, the method of the present invention includes any one or more of the above referenced embodiments of the third aspect of the present invention wherein the step of forming a film is performed by blade coating, solvent coating, lamination, spray coating, extruding, injection molding, or a combination thereof.

In one or more embodiments, the method of the present invention includes any one or more of the above referenced embodiments of the third aspect of the present invention wherein the step of forming a film comprises: preparing a blade coating apparatus comprising a base, a casting blade box, a solution well, and an adjustable doctor blade, wherein the adjustable doctor blade may be adjusted up to increase the thickness of a film being formed in the blade coating apparatus or down to decrease the thickness of a film being formed in the blade coating apparatus and the casting blade box is free to move along a top surface of the base; placing the substrate between the casting blade box and the base; placing the degradable amino acid based poly(ester urea) polymer and drug solution in the solution well of the blade coating apparatus; and moving the casting blade box across an upper surface of the substrate to form a film comprising the degradable amino acid based poly(ester urea) polymer and the drug.

In one or more embodiments, the method of the present invention includes any one or more of the above referenced embodiments of the third aspect of the present invention wherein the step of preparing a blade coating apparatus further comprising adjusting the adjustable doctor blade up or down to form films having a desired thickness. In one or more embodiments, the method of the present invention includes any one or more of the above referenced embodiments of the third aspect of the present invention wherein the blade coating apparatus is adjusted to form films having a thickness of from 1µm to 100 µm, preferably from 2 µm to 500 µm, and more preferably from 10 µm to 150 µm. In one or more of these embodiments, the blade coating apparatus is adjusted to form films having a thickness of from 40 µm to 140 µm.

### BRIEF DESCRIPTION OF THE DRAWINGS

For a more complete understanding of the features and advantages of the present invention, reference is now made to the detailed description of the invention along with the accompanying figures in which:

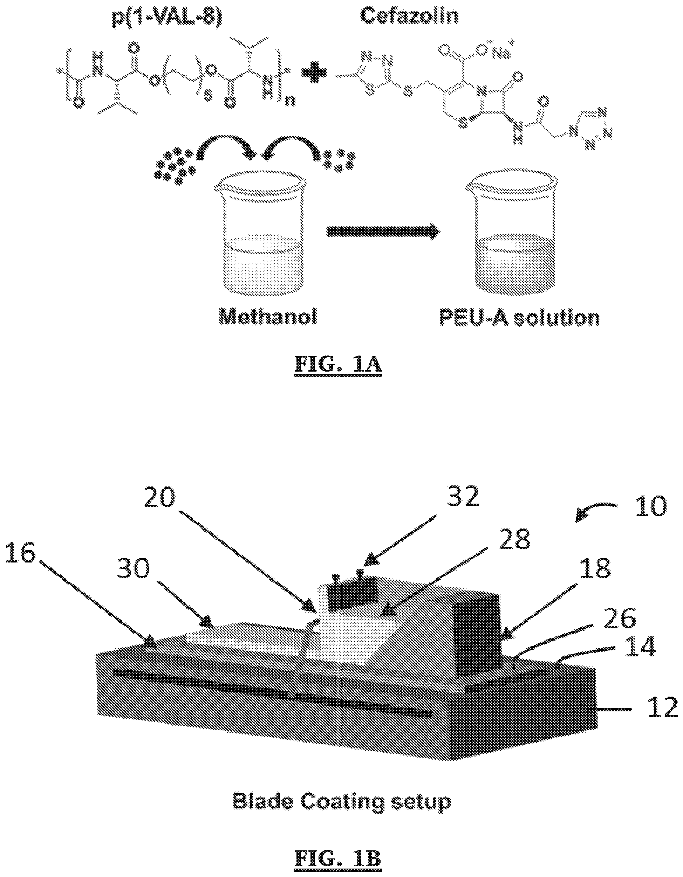

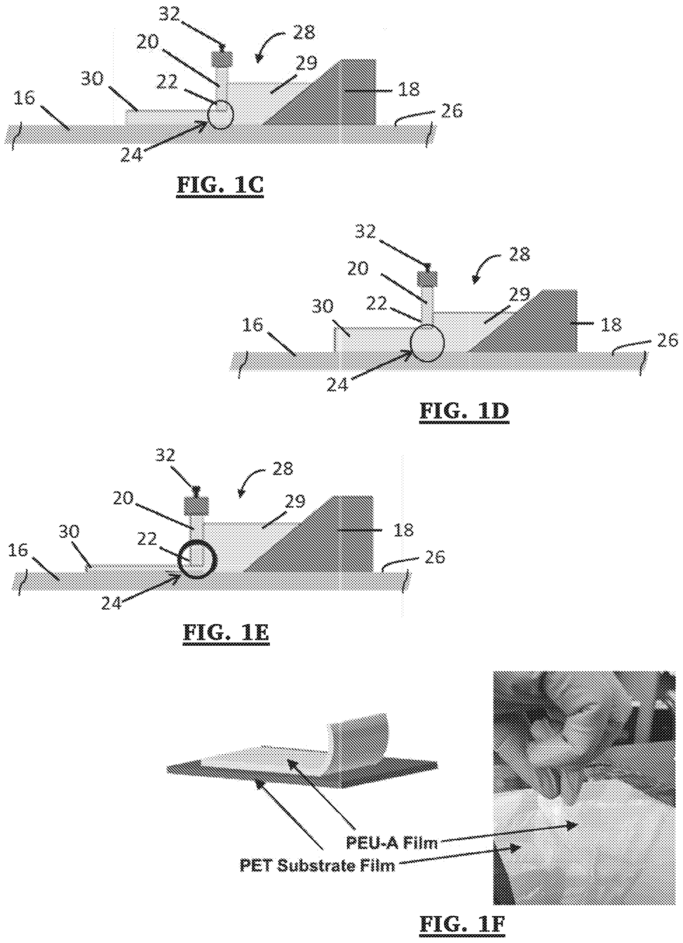

FIGS. 1A-F are schematic diagrams and images showing steps in preparing stages in the preparation of PEU films. FIG. 1A is a schematic diagram showing preparation of the PEU-Antibiotic (PEU-A) solutions. PEU-Antibiotic (PEU-A) solutions containing 2%, 5% and 10% of Cefazolin by weight (w.r.t. PEU) were prepared. FIG. 1B is a schematic diagram showing the apparatus used for the blade assisted solution casting. The PEU-A solution was poured into the well of casting blade box. As the casting blade moves, the solution flows through the gap between the casting blade and substrate. The substrate gets coated with PEU-A solution which forms a uniform film. FIGS. 1C-E are schematic diagrams showing mechanism for obtaining different film thicknesses using the casting blade box shown in FIG. 1B Target thickness for the film can be tuned by changing the gap height. FIG. 1F is a schematic drawing and image showing removal of the PEU A film from the substrate. The PEU films did not adhere strongly to the substrate film and could be readily peeled off.
FIG. 2 is a ¹H-NMR of di-p-toluenesulfonic acid salt of bis-(L-valine)-1,8-octyl diester. ¹H-NMR (300 MHz, DMSO-*d*₆, 30 °C) δ ppm 0.97 (dd, 12H) 1.27 (s, 8H) 1.58 (m, 4H) 2.13 (m, 2H) 2.27 (s, 6H), 3.90 (d, 2H) 4.12-4.16 (m, 4H) 7.09-7.12 (d, 4H) 7.45-48 (d, 4H) 8.28 (s, 6H).
FIG. 3 is a ¹H-NMR spectrum for the L-valine-based poly(ester urea) p(1-Val-8). ¹H-NMR (300 MHz, DMSO-*d*₆, 30 °C) δ ppm 0.83 (dd, 12H) 1.25 (s, 12H) 1.53 (s, 4H) 1.98 (m, 2H) 4.03 (m, 2H) 4.10 (m, 4H) 6.37 (d, 2H).
FIG. 4 is the calibration curve for the antibiotic Cefazolin sodium, using a DAD detector at λ= 270 nm.

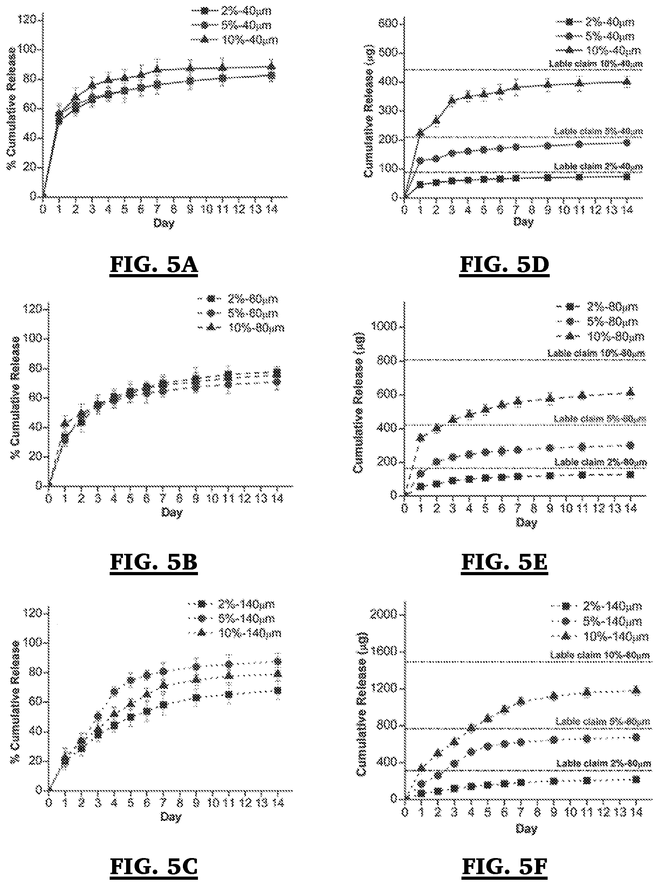

FIGS. 5A-F are graphs showing the results of *in vitro* antibiotic release experiments. FIGS. 5A-C show *In vitro* percent cumulative release for 2%-40µm, 2%-80 µm, 2%-140 µm, 5%-40 µm, 5%-80 µm, 5%-140 µm, 10%-40 µm, 10%-80 µm, and 10%-140 µm samples. The percentage of cumulative antibiotic released at each timepoint was calculated based on label claim. Label claim 100% of antibiotic in a PEU disk which was determined from content uniformity testing. FIGS. 5D-F show *In vitro* cumulative release profiles for 2%-40µm, 2%-80 µm, 2%-140 µm, 5%-40 µm, 5%-80 µm, 5%-140 µm, 10%-40 µm , 10%-80 µm, and 10%-140 µm samples. The amount of antibiotic released was determined using HPLC analysis. Samples were placed in PBS (3 mL) at 37 °C, and the sink was replaced at each timepoint. (n = 5 samples per group).
FIGS. 6A-C are cummulative percent drug release plots showing fitting into the Higuchi model with their fitting equation, and R²-Values for 2%-40µm (FIG. 6A), 2%-80 µm (FIG. 6B), and 2%-140 µm (FIG. 6C) samples.
FIGS. 7A-C are cummulative percent drug release plots showing fitting into the Higuchi model with their fitting equation, and R²-Values for 5%-40 µm (FIG. 7A), 5%-80 µm (FIG. 7B), and 5%-140 µm (FIG. 7C) samples.
FIGS. 8A-C are cummulative percent drug release plots showing fitting into the Higuchi model with their fitting equation, and R²-Values for 10%-40 µm (FIG. 8A), 10%-80 µm (FIG. 8B), and 10%-140 µm (FIG. 8C) samples.

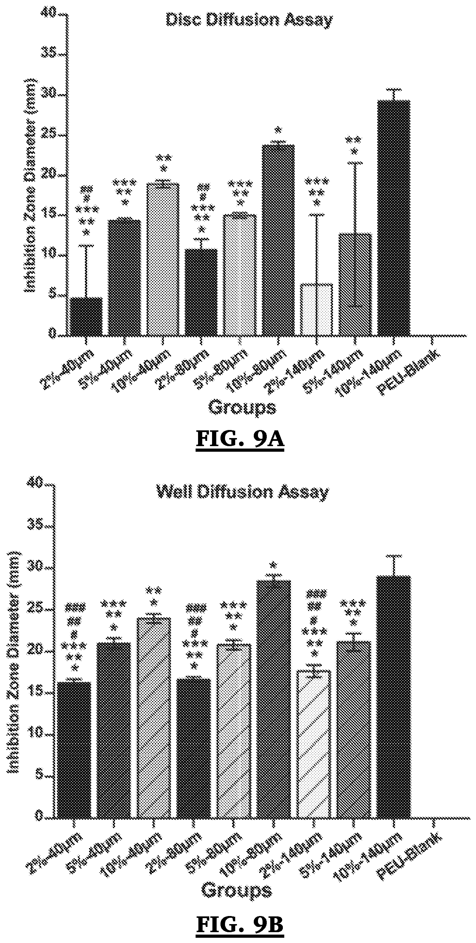

FIGS. 9A-B are graphs showing the results of *In vitro* antimicrobial susceptibility testing using disk-diffusion (FIG. 9A) and well-diffusion (FIG. 9B) assays. To determine the diameters of inhibition zone measurements were done in four perpendicular directions on each sample and the mean values were calculated. (n=3 samples per group). In FIG. 9A (Disc Diffusion Assays), the groups marked with * had significantly lower CFU when compared to group 10%-140µm. Similarly, groups marked with **, ***, #, ## had significantly lower CFU in their wells when compared to 10%-80µm, 10%-40µm, 5%-80µm and 5%-40µm respectively. In FIG. 9B (Well Diffusion Assays), the groups marked with *, **, ***, #, ##, ### again had significantly lower CFU in their wells when compared to 10%-140µm, 10%-80µm, 10%-40µm, 5%-140µm, 5%-80µm and 5%-40µm respectively. Tukey's test was used for means comparison (P=0.05), the homogeneity of variance was tested by Levene's test.
FIGS. 10A-B are images showing the results for the disk-diffusion (FIG. 10A) and well-diffusion assays (FIG. 10B) done with Blank p(1-VAL-8) film without any antibiotic-loaded in them. The absence of inhibition zone indicates that p(1-VAL-8) does not possess inherent antimicrobial properties.

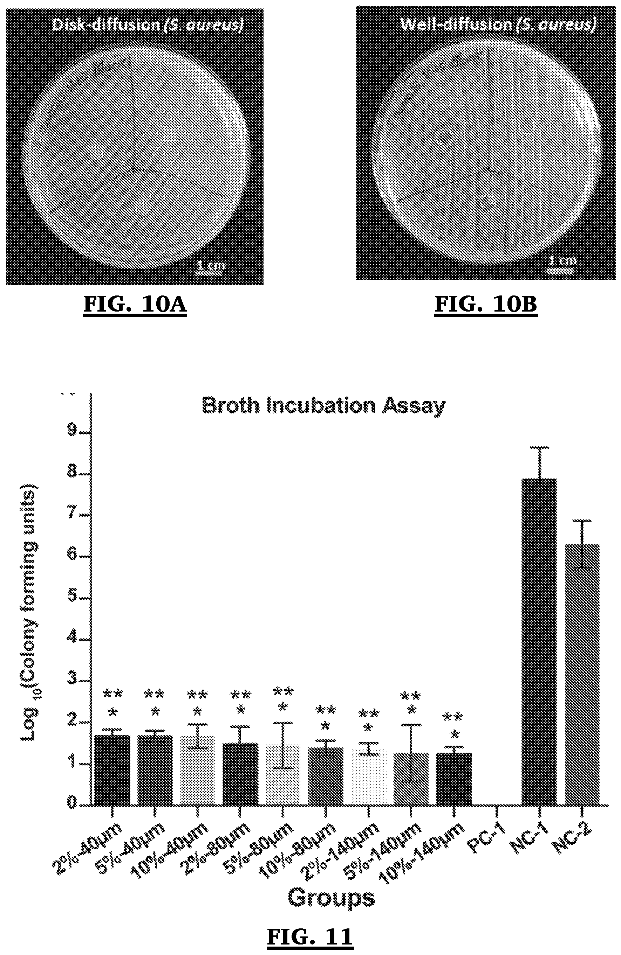

FIG. 11 is a graphs showing the results of a broth incubation assay showing bacteria colony forming units (CFU) present in wells corresponding to PEU-A film groups and controls (n=3). The groups marked with * had significantly lower CFU when compared to Negative control-1 (NC-1). The groups marked with ** had significantly lower CFU in their wells when compared to Negative control-2 (NC-2). All sample groups had significantly lower CFU when compared to both negative controls NC-1, NC-2. Within the groups the CFU differences were not significant. Tukey's test was used for means comparison (P=0.05), the homogeneity of variance was tested by Levene's test.
FIGS. 12A-I are images showing the results of the disc-diffusion assays for all 9 groups of PEU-A films.
FIGS. 13A-I are images showing the results of the well-diffusion assays for all 9 groups of PEU-A films.
FIG. 14 is an ATR-FTIR spectrum of the p(1-VAL-8) PEU. The IR bands highlighted in the figure are distinctive bands that demonstrate successful synthesis of the p(1-Val-8) PEU. The band highlighted in blue indicate C=O stretching from the urea bond and the red highlighted band indicates C=O stretching from the ester bond.
FIG. 15 is a DSC curve of the p(1-VAL-8) PEU. The curve shows a partial portion of the second heating cycle in a DSC trace (exotherm up) for p(1-VAL-8) PEU. The *T*_{g} calculated using the curve above was 52.4 °C.
FIG. 16 is a TGA curve of the p(1-VAL-8) PEU. The *T*_{d} calculated using the curve above was 297 °C.
FIG. 17 is a SEC chromatogram of the p(1-VAL-8) PEU. The molecular mass values calculated using the EcoSEC software, *M*_{w}= 52,900 g/mol, *M*ₙ = 28,400 g/mol and *D*ₘ = 1.86.

### DETAILED DESCRIPTION OF THE ILLUSTRATIVE EMBODIMENTS

As set forth above, implant-associated infections acquired during implantation procedures are a major complication prove challenging to treat therapeutically resulting in chronic and fatal infections. Cardiac implantable electronic device (CIED) infections are one such type. The conventional, systemic antibiotic delivery is less efficacious in many of these cases and leaves much to be desired. Localized antibiotic delivery could be employed to supplement for systemic administration shortcomings. In various embodiments, the present invention is directed to a drug-loaded amino acid based poly(ester urea) film that produces localized drug delivery for implanted devices. In some embodiments, the present invention is directed to an antibiotic-loaded L-Valine poly(ester urea) film for localized antibiotic delivery for CIEDs or other devices during implantation. Thickness and loading concentration influence the amount and rate of antibiotic release. This dependence of release on thickness and loading concentration gives a handle to fabricate PEU-A films with any desired release profile that can deliver the therapeutically relevant amount of antibiotic.

The following terms may have meanings ascribed to them below, unless specified otherwise. In addition, the materials, methods, and examples are illustrative only and not intended to be limiting. As used herein, the singular forms "a", "and", and "the" include plural references unless the context clearly dictates otherwise. All technical and scientific terms used herein have the same meaning.

Unless specifically stated or obvious from context, as used herein, the term "about" is understood as within a range of normal tolerance in the art, for example within 2 standard deviations of the mean. "About" can be understood as within 10%, 9%, 8%, 7%, 6%, 5%, 4%, 3%, 2%, 1%, 0.5%, 0.1%, 0.05%, 0.01% or 0.001% of the stated value. Unless otherwise clear from context, all numerical values provided herein in the specification and the claim can be modified by the term "about." Further, ranges provided herein are understood to be shorthand for all of the values within the range. For example, a range of 1 to 50 is understood to include any number, combination of numbers, or sub-range from the group consisting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50.

In a first aspect, the present invention is directed to a drug-loaded degradable amino acid based poly(ester urea) film for drug delivery comprising an amino acid based poly(ester urea) polymer and a drug. In one or more of these embodiments, the amino acid based poly(ester urea) polymer used to form the degradable amino acid based poly(ester urea) film will comprise residues of two or more amino acid based diester or multi-ester monomers, forming amino acid based diester or multi-ester monomers segments that are joined by urea linkages. In various embodiments, these urea linkages will comprise a carbonyl group (C=O) bonded to the terminal amine groups of two amino acid based diester or multi ester monomers, thereby forming a urea linkage (urea bond) connecting the two amino acid based diester or multi ester monomers.

As used herein, the terms "degradable," and "biodegradable" are used interchangeably to refer to a macromolecule or other polymeric substance susceptible to degradation by biological activity by lowering the molecular masses of the macromolecules that form the substance. The PEU polymers used to form the drug-loaded degradable amino acid based poly(ester urea)s of various embodiments of the present invention are degradable. Their specific degradation properties will depend upon such things as the molecular weight of the PEU, the size and structure of the diol or polyols residue used to form the monomers, and the particular amino acid residue(s) present. By manipulating these variables, the degradation properties of the drug-loaded degradable amino acid based poly(ester urea)s of the present invention may be tuned to obtain a wide variety degradation profiles and other characteristics and may be tuned for specific applications.

As used herein, the term "residue(s)" is used to refer generally to the part of a monomer or other chemical unit that has been incorporated into a polymer or large molecule. As follows, the terms "amino acid based diester monomer residue," "amino acid based multi-ester monomer residue," and "residue of" the amino acid based diester or multi-ester monomer all refer to the portion of the particular monomer starting material used that has been incorporated into the amino acid based diester or multi-ester monomer segments and by extension, into the PEU polymer. Similarly, the an "amino acid residue" or "residue of" a particular amino acid refer to the portion of that amino acid that has been incorporated into the amino acid based diester or multi-ester monomer starting materials or segments.

In one or more embodiments, the amino acid based diester monomer segments may comprise the residues of two amino acids separated by from 1 to 20 carbon atoms. In some of these embodiments, the two amino acid residues forming the amino acid based diester monomer segments may be residues of the same amino acid, but this need not be the case and embodiments where the two amino acid residues forming the amino acid based diester monomer segments are within the scope of the invention. In some embodiments, the two amino acid residues forming the amino acid based diester monomer segments may each be, without limitation, residues of alanine (ala - A); arginine (arg - R); asparagine (asn - N); aspartic acid (asp - D); cysteine (cys - C); glutamine (gln - Q); glutamic acid (glu - E); glycine (gly - G); histidine (his - H); isoleucine (ile - I); leucine (leu - L); lysine (lys - K); methionine (met - M); phenylalanine (phe - F); serine (ser - S); threonine (thr - T); tryptophan (trp - W); tyrosine (tyr - Y); valine (val - V) or a combinations thereof. In some embodiments, the amino acid based diester monomer segments may comprise the residues of two L-serine molecules, separated by from 1 to 20 carbon atoms. In some embodiments, the hydroxyl groups will be separated by from 2 to 20, in other embodiments, from 6 to 20, in other embodiments, from 10 to 20, in other embodiments, from 12 to 20, in other embodiments, from 1 to 17, in other embodiments, from 1 to 13, in other embodiments, from 1 to 9 carbon atoms.

In some embodiments, the amino acid based diester monomer segments are the reaction product of two amino acids and a C1-C20 diol. In some of these embodiments, the two amino acids may each be, without limitation, residues of alanine (ala - A ); arginine (arg - R); asparagine (asn - N); aspartic acid (asp - D); cysteine (cys - C); glutamine (gln - Q); glutamic acid (glu - E); glycine (gly - G); histidine (his - H); isoleucine (ile - I); leucine (leu - L); lysine (lys - K); methionine (met - M); phenylalanine (phe - F); serine (ser - S); threonine (thr - T); tryptophan (trp - W); tyrosine (tyr - Y); valine (val - V) or combinations thereof. Again, in some of these embodiments, the two amino acids may be the same or different. In some of these embodiments, the two amino acids are both L-valine.

The C1-C20 diol in these embodiments is not particularly limited and will comprise two reactive hydroxyl groups separated by from 1 to 20 carbon atoms. In some embodiments, the hydroxyl groups will be separated by from 2 to 20, in other embodiments, from 6 to 20, in other embodiments, from 10 to 20, in other embodiments, from 12 to 20, in other embodiments, from 1 to 17, in other embodiments, from 1 to 13, in other embodiments, from 1 to 9 carbon atoms. In various embodiments, suitable diols may include, without limitation, 1,6-hexanediol, 1,8-octanediol, 1,9-nonanediol, 1,10-decanediol, 1,11-undecanediol, 1,12-dodecanediol, 1,13-tridecanediol, 1,14-tetradecanediol, 1,15-pentadecanediol, 1,16-hexadecanediol, 1,17-heptadecanediol, 1,18-octadecanediol, 1,19-nonadecanediol, 1,20-icosanediol, 2-butene-1,4-diol, 3,4-dihydroxy-1-butene, 7-octene-1,2-diol, 3-hexene-1,6-diol, 1,4-butynediol, trimethylolpropane allyl ether, 3-allyloxy-1,2-propanediol, 2,4-hexadiyne-1,6-diol, 2-hydroxymethyl-1,3-propanediol, or combinations thereof. In some embodiments, the C1-C20 diol will be 1,8-octanediol.

In one or more embodiments, the two or more amino acid based diester monomer segments forming the amino acid based PEU polymer will have the formula: 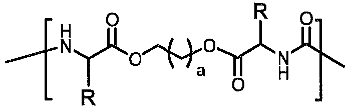 wherein R is -CH₃, -(CH₂)₃NHC(NH₂)C=NH, -CH₂CONH₂,-CH₂COOH, -CH₂SH, -(CH₂)₂COOH, -(CH₂)₂CONH₂, -NH₂, -CH₂C=CH-N=CH-NH, -CH(CH₃)CH₂CH₃, -CH₂CH(CH₃)₂, -(CH₂)₄NH₂, -(CH₂)₂SCH₃, -CH₂Ph, -CH₂OH, -CH(OH)CH₃, -CH₂-C=CH-NH-Ph, -CH₂-Ph-OH,-CH(CH₃)₂, or -CH₂C₆H₄OCH₂C₆H₅ and a is an integer from 1 to 20.

In one or more embodiments, the degradable amino acid based PEU polymer will have the formula: 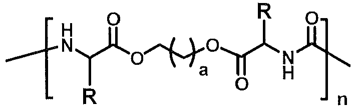 wherein R is -CH₃, -(CH₂)₃NHC(NH₂)C=NH, -CH₂CONH₂,-CH₂COOH, -CH₂SH, -(CH₂)₂COOH, -(CH₂)₂CONH₂, -NH₂, -CH₂C=CH-N=CH-NH, -CH(CH₃)CH₂CH₃, -CH₂CH(CH₃)₂, -(CH₂)₄NH₂, -(CH₂)₂SCH₃, -CH₂Ph, -CH₂OH, -CH(OH)CH₃, -CH₂-C=CH-NH-Ph, -CH₂-Ph-OH,-CH(CH₃)₂, or -CH₂C₆H₄OCH₂C₆H₅; a is an integer from 1 to 20; and n is an integer from 20 to 300.

In some of these embodiments, a may be an integer from 1 to 18, in other embodiments, from 1 to 16, in other embodiments, from 1 to 12, in other embodiments, from 1 to 8, in other embodiments, from 1 to 4, in other embodiments, from 5 to 20, in other embodiments, from 9 to 20, in other embodiments, from 13 to 20, in other embodiments, from 15 to 20. In some embodiments, n may be an integer from 20 to 200, in other embodiments, from 20 to 160, in other embodiments, from 20 to 120, in other embodiments, from 20 to 80, in other embodiments, from 20 to 40, in other embodiments, from 50 to 300, in other embodiments, from 100 to 300, in other embodiments, from 150 to 300, in other embodiments, from 200 to 300. Here, as well as elsewhere in the specification and claims, individual range values can be combined to form additional non-disclosed ranges.

In some embodiments, the amino acid based diester monomers forming the amino acid based PEU are the reaction product of two L-valine molecules and a C1-C20 diol. In one or more of these embodiments, the degradable amino acid based PEU polymers used to form the drug-loaded amino acid based PEU films of the present invention have the formula: 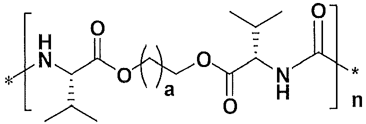 where a is an integer from 1 to 20 and n is an integer from 20 to 300. In some embodiments, a may be an integer from 1 to 18, in other embodiments, from 1 to 16, in other embodiments, from 1 to 12, in other embodiments, from 1 to 8, in other embodiments, from 1 to 4, in other embodiments, from 5 to 20, in other embodiments, from 9 to 20, in other embodiments, from 13 to 20, in other embodiments, from 15 to 20. In some embodiments, n may be an integer from 20 to 200, in other embodiments, from 20 to 160, in other embodiments, from 20 to 120, in other embodiments, from 20 to 80, in other embodiments, from 20 to 40, in other embodiments, from 50 to 300, in other embodiments, from 100 to 300, in other embodiments, from 150 to 300, in other embodiments, from 200 to 300. Here, as well as elsewhere in the specification and claims, individual range values can be combined to form additional non-disclosed ranges.

In one or more embodiments, the amino acid based poly(ester urea) polymer used to form the degradable amino acid based poly(ester urea) films of the present invention will have a number average molecular mass (Mₙ) of from 2000 Da to 80,000 Da. In some embodiments, the number average molecular mass (Mₙ) may be from 2000 Da to 70,000 Da, in other embodiments, from 2000 Da to 50,000 Da, in other embodiments, from 2000 Da to 30,000 Da, in other embodiments, from 10,000 Da to 80,000 Da, in other embodiments, from 30,000 Da to 80,000 Da, and in other embodiments, from 50,000 Da to 80,000 Da. In some embodiments, the amino acid based poly(ester urea) polymer used to form the degradable amino acid based poly(ester urea) films of the present invention will have a number average molecular mass (Mₙ) of from 20,000 Da to 40,000 Da.

In some other embodiments, the amino acid based poly(ester urea) polymer used to form the degradable amino acid based poly(ester urea) films of the present invention may also be branched. In one or more embodiments, amino acid based poly(ester urea) polymer will comprise one or more of the amino acid based multi-ester monomer segments. In various embodiments, these amino acid based multi-ester monomer segments will comprise three or more amino acid residues, each separated by from the others by from 2 to 20 carbon atoms.

As set forth above, the drug-loaded degradable amino acid based poly(ester urea) films of the present invention also comprises a drug or other bioactive substance. The type of drug that may be used is not particularly limited and may include, without limitation, antibiotics, cancer drugs, antipsychotics, antidepressants, sleep aids, tranquillizers, anti-Parkinson's drugs, mood stabilizers, pain killers, anti-inflammatories, antimicrobials, and combinations thereof. In some embodiments, drug may be an antibiotic including, but not limited to, lipopeptides, fluoroquinolone, lipoglycopeptides, cephalosporins, penicillins, monobactams, carbapenems, macrolide antibiotics, lincosamides, streptogramins, aminoglycoside antibiotics, quinolone antibiotics, sulfonamides, tetracycline antibiotics, chloraphenicol, metronidazole, tinidazole, nitrofurantoin, glycopeptides, oxazolidinones, rifamycins, polypeptides, tuberactinomycins, or a combinations or pharmaceutically acceptable salts thereof. In some embodiments, the drug may be cefazolin sodium or a pharmaceutically acceptable salt thereof.

In various embodiments, the drug is substantially uniformly distributed throughout said degradable amino acid based poly(ester urea) polymer. This need not, however, be the case and other embodiments where the drug is not substantially uniformly distributed are possible.

In one or more embodiments, the drug-loaded degradable amino acid based PEU film of the present invention may comprise from 1 weight percent to 50 weight percent, preferably from 1 weight percent to 10 weight percent, and more preferably from 1 weight percent to 5 weight percent of said drug. In some embodiments, drug-loaded degradable amino acid based poly(ester urea) film of the present invention will comprise from 1 weight percent to 30 weight percent, in other embodiments, from 1 weight percent to 20 weight percent, in other embodiments, from 1 weight percent to 10 weight percent, in other embodiments, from 1 weight percent to 5 weight percent, in other embodiments, from 3 weight percent to 50 weight percent, in other embodiments, from 12 weight percent to 50 weight percent, in other embodiments, from 25 weight percent to 50 weight percent, and in other embodiments, from 35 weight percent to 50 weight percent drug.. In some embodiments, degradable amino acid based poly(ester urea) film comprises from 2 weight percent to 10 weight percent of said drug. Here, as well as elsewhere in the specification and claims, individual range values can be combined to form additional non-disclosed ranges.

In various embodiments, the drug-loaded degradable amino acid based poly(ester urea) film of the present invention will have a thickness of from 1µm to 1000 µm, preferably from 10 µm to 500 µm, and more preferably from 10 µm to 150 µm. In some embodiments, the drug-loaded degradable amino acid based poly(ester urea) film of the present invention will have a thickness of from 1 µm to 800 µm, in other embodiments, from 1 µm to 500 µm, in other embodiments, from 1 µm to 200 µm, in other embodiments, from 1 µm to 100 µm, in other embodiments, from 30 µm to 1000 µm, in other embodiments, from 150 µm to 1000 µm, in other embodiments, from 450 µm to 1000 µm, in other embodiments, from 750 µm to 1000 µm. In some embodiments, the degradable amino acid based poly(ester urea) film will have a thickness of from 40 µm to 140 µm. Here, as well as elsewhere in the specification and claims, individual range values can be combined to form additional non-disclosed ranges.

In a second aspect, the present invention is directed to an implantable medical device comprising the drug-loaded degradable amino acid based poly(ester urea) film described above. The type of implantable medical device is not particularly limited and may be any device, structure or material that is inserted into the body of a patient to treat a medical condition, or for any other purpose. Examples of suitable implantable medical devices may include, without limitation, cardiac implantable electronic devices (CIEDs), pace makers, implantable cardioverter defibrillators (ICDs), artificial heart valves, surgical mesh, hernia mesh films, transvaginal mesh, transabdominal mesh, sutures, shunts, stents, adhesion barriers, tissue scaffolds, auditory implants, ear tubes, cochlear implants, neurological implants, neural implants, implantable gastric stimulators, diaphragmatic/phrenic nerve stimulators, orthopedic implants, joint replacement appliances, spinal fusion hardware, surgical hardware for fracture repair (metal plates, pins, screws, rods, etc.), contraceptive implants, intra-uterine devices (IUDs), or cosmetic implants, implantable prostheses, and combinations thereof. In one or more embodiments, the implantable medical device of the present invention comprises a cardiac implantable electronic device (CIED).

In various embodiments, the drug-loaded degradable amino acid based poly(ester urea) film described above may be applied directly to the implantable medical device by any suitable method known in the art for that purpose, including, but not limited to, blade coating, lamination, dip coating, solvent coating, spray coating, extrusion, injection molding, or a combination thereof. In some embodiments, the drug-loaded degradable amino acid based poly(ester urea) film described above may be applied to the implantable medical device to be inserted in the body by blade coating, as described below.

In some other embodiments, the implantable medical device may be wrapped in the drug-loaded degradable amino acid based poly(ester urea) film described above prior to or during implantation. In some embodiments, the implantable medical device may be placed in a pocket or envelope formed from the drug-loaded degradable amino acid based poly(ester urea) film described above prior to or during implantation. In some embodiments, the implantable medical device may be coated or surrounded on all sides by the drug-loaded degradable amino acid based poly(ester urea) film described above, but this need not the case. Embodiments where the implantable medical device is only partially coated or surrounded by the drug-loaded degradable amino acid based poly(ester urea) film described above are contemplated and within the scope of the present invention.

In a third aspect, the present invention is directed to a method of making the drug-loaded degradable amino acid based PEU film for drug delivery described above comprising: preparing a suitable substrate; preparing a degradable amino acid based
PEU polymer; selecting a drug to be delivered and dissolving it in a co-solvent for the drug and the degradable amino acid based poly(ester urea) polymer; dissolving the degradable amino acid based poly(ester urea) polymer in the drug solution; and forming the amino acid based poly(ester urea) polymer and drug solution into a film on the substrate. The film is then allowed to dry and, in some embodiments, separating from the substrate as a free standing film.

First, the particular degradable amino acid based PEU polymer to be used is selected and prepared. In various embodiments, any of the degradable amino acid based PEU polymers described above may be selected for use in this method. The degradable amino acid based EU polymer used to form the drug-loaded degradable amino acid based poly(ester urea) film of the present invention may be formed by any method known in the art for that purpose. In some embodiments, the degradable amino acid based poly(ester urea) polymer used to form the drug-loaded degradable amino acid based poly(ester urea) film of the present invention may be formed as shown in Examples 1-16, below.

Generally in these methods, the degradable amino acid based poly(ester urea) polymer is formed in a two-step process wherein two or more C1-C20 diols or C3-C60 polyol are reacted with stoichiometrically appropriate quantity of amino acids in the presence of an acid to produce counter ion protected amino acid based diester or multi-ester monomers. These monomers are then linked together by interfacial polymerization with a urea bond forming material to form the degradable amino acid based poly(ester urea) polymer. As used herein, the term "interfacial polymerization" refers to polymerization that takes place at or near the interfacial boundary of two immiscible fluids. As used herein, the terms "PEU forming compound" and "PEU forming material"
are used interchangeably to refer to a material capable of placing a carboxyl group between two amine groups, thereby forming a urea bond. Suitable PEU forming material may include, without limitation, triphosgene, diphosgene, or phosgene.

In some of these embodiments, the counter ion protected amino acid based diester monomer is synthesized *via* Fischer esterification between a C1 to C20 diol and a suitable amino acid, which has been protonated using an acid, such as HCl or *p-*toluenesulfonic acid, to prevent transamidation and exchange reactions (see, e.g., Schemes 1 and 2, below). As will be apparent to those of skill in the art, steps should be taken to prevent transamidation of the ester bonds on the diester prior to or during polymerization. In some embodiments, transamidation may be prevented or limited by protecting the amine groups on the amino acid-based diester monomers being formed with one or more counter-ions. Accordingly, in these embodiments, a suitable acid or other source of counter-ions is added to the solution prior to or during formation of the diester monomer. In the embodiment shown in Schemes 1 and 2, below, for example, the amine groups on the amino acids were protonated using p-toluenesulfonic acid to prevent transamidation and exchange reactions, but the invention is not so limited. One of ordinary skill in the art will be able to select a suitable counter-ion without undue experimentation. Materials capable of producing suitable amine protecting counter-ions may include without limitation, p-toluene sulfonic acid monohydrate, chlorides, bromides, acetates, trifloroacetate, and combinations thereof. In some embodiments, the acid used may be p-toluene sulfonic acid monohydrate. In some embodiments, the acid used may be HCl.

In one or more of these embodiments, the selected C1-C20 diol, the selected amino acids and an acid, such as p-toluenesulfonic acid monohydrate or HCl, are dissolved in a suitable co-solvent, such as toluene, in a suitable reaction vessel equipped with a Dean-Stark Trap and a magnetic stirrer. The solution is then heated to reflux for from about 2 to about 24 hours or until the reaction is substantially complete. The vessel is then allowed to return to ambient temperature and the counter ion protected amino acid based diester monomer may be isolated, collected and purified using methods known in the art for that purpose. In some embodiments, the counter ion protected amino acid based diester monomer may be isolated by vacuum filtration using a Buchner funnel and purified through recrystallization by dissolving it in boiling water, vacuum filtering hot, and cooling to room temperature to obtain the counter ion protected amino acid based diester monomer as a white solid precipitate.

In some embodiments, the amino acids used to form the amino acid based diester monomer segments may include, without limitation, alanine (ala - A ); arginine (arg - R); asparagine (asn - N); aspartic acid (asp - D); cysteine (cys - C); glutamine (gln - Q); glutamic acid (glu - E); glycine (gly - G); histidine (his - H); isoleucine (ile - I); leucine (leu - L); lysine (lys - K); methionine (met - M); phenylalanine (phe - F); serine (ser - S); threonine (thr - T); tryptophan (trp - W); tyrosine (tyr - Y); valine (val - V) and combinations thereof. In some of these embodiments, only one amino acid is used form the amino acid based diester monomer segments, but this need not be the case. In some other embodiments, two or more different amino acids are used. In some embodiments, the amino acid used to form based diester monomer segments will be *L*-serine. In some embodiments, the amino acid used to form based diester monomer segments will be *L*-valine.

The C1-C20 diol or diols used to form the amino acid based diester monomer segments are not particularly limited and may be any compound containing two reactive hydroxyl groups separated by from 1 to 20 carbon atoms, provided that any other functional groups are protected. In some embodiments, the hydroxyl groups in these compounds will be separated by from 2 to 20, in other embodiments, from 6 to 10, in other embodiments, from 10 to 20, in other embodiments, from 12 to 20, in other embodiments, from 1 to 17, in other embodiments, from 1 to 13, in other embodiments, from 1 to 9 carbon atoms. Here, as well as elsewhere in the specification and claims, individual range values can be combined to form additional non-disclosed ranges.

In various embodiments, suitable diols may include, without limitation, 1,6-hexanediol, 1,8-octanediol, 1,9-nonanediol, 1,10-decanediol, 1,11-undecanediol, 1,12-dodecanediol, 1,13-tridecanediol, 1,14-tetradecanediol, 1,15-pentadecanediol, 1,16-hexadecanediol, 1,17-heptadecanediol, 1,18-octadecanediol, 1,19-nonadecanediol, 1,20-icosanediol, 2-butene-1,4-diol, 3,4-dihydroxy-1-butene, 7-octene-1,2-diol, 3-hexene-1,6-diol, 1,4-butynediol, trimethylolpropane allyl ether, 3-allyloxy-1,2-propanediol, 2,4-hexadiyne-1,6-diol, 2-hydroxymethyl-1,3-propanediol, or combinations thereof. In some embodiments, the C1-C20 diol will be 1,8-octanediol.

In one or more embodiments, the counter ion protected amino acid based diester monomer will have the formula: 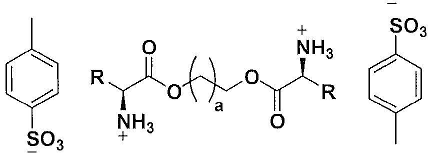 where each R is -CH₃, -(CH₂)₃NHC(NH₂)C=NH, -CH₂CONH₂,-CH₂COOH, -CH₂SH, - (CH₂)₂COOH, -(CH₂)₂CONH₂, -NH₂, -CH₂C=CH-N=CH-NH, -CH(CH₃)CH₂CH₃, -CH₂CH(CH₃)₂, -(CH₂)₄NH₂, -(CH₂)₂SCH₃, -CH₂Ph, -CH₂OH, -CH(OH)CH₃, -CH₂-C=CH-NH-Ph, -CH₂-Ph-OH,-CH(CH₃)₂, or -CH₂C₆H₄OCH₂C₆H₅ and a is an integer from 1 to 20. In various embodiments, the ranges for a may be as set forth above.

In one or more embodiments, the counter ion protected amino acid based diester monomer will have the formula: 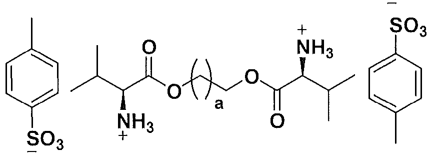 where a is an integer from 1 to 20. In various embodiments, the ranges for a may be as set forth above.

As set forth above, in a second step these counter ion protected amino acid based diester monomers are linked together using urea linkages by the interfacial polymerization of these counter ion protected amino acid based diester monomers with a urea bond forming material, such as phosgene, diphosgene, or triphosgene, to provide the degradable amino acid based poly(ester urea) polymers used to form the drug-loaded degradable amino acid based poly(ester urea) films of the present invention. In some embodiments, the interfacial polymerization of the counter ion protected amino acid based diester monomers is performed by dissolving the monomers and a base, such as sodium carbonate, in a cosolvent such as distilled water, in a suitable reaction vessel, such as a 3-neck round-bottom flask. In one or more embodiments, the base is an inorganic base. The solution is placed in a water bath (approx. 40 °C) or other suitable heat sink, stirred until clear, and then cooled to 0 °C. In a separate container, additional base (e.g., sodium carbonate) is dissolved in distilled water, added to the reaction vessel, and allowed to stir until clear. Separately, the urea bond forming material (e.g., triphosgene) is dissolved in distilled in a suitable solvent, such as chloroform, and then added to the reaction flask. The solution as allowed to stir for 30 min and an additional aliquot of the urea bond forming material (e.g., triphosgene) dissolved in distilled chloroform is slowly added to the solution. The reaction was stirred for from 2 h to 100 h to produce the degradable amino acid based poly(ester urea) polymer, which may be isolated, collected and purified using methods known in the art for that purpose. Here, as well as elsewhere in the specification and claims, individual range values can be combined to form additional non-disclosed ranges.

In some embodiments, the counter ion protected amino acid based diester monomers are linked to form the amino acid based PEU polymers described above.

In one or more embodiments, the amino acid based PEU polymer used to form the drug loaded PEU film will have the formula: 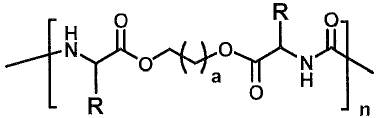 where each R is -CH₃, -(CH₂)₃NHC(NH₂)C=NH, -CH₂CONH₂,-CH₂COOH, -CH₂SH, - (CH₂)₂COOH, -(CH₂)₂CONH₂, -NH₂, -CH₂C=CH-N=CH-NH, -CH(CH₃)CH₂CH₃, -CH₂CH(CH₃)₂, -(CH₂)₄NH₂, -(CH₂)₂SCH₃, -CH₂Ph, -CH₂OH, -CH(OH)CH₃, -CH₂-C=CH-
NH-Ph, -CH₂-Ph-OH,-CH(CH₃)₂, or -CH₂C₆H₄OCH₂C₆H₅; a is an integer from 1 to 20; and n is an integer from 20 to 300. In some embodiments, the ranges for a and n may be as set forth above.

In one or more embodiments, the amino acid based PEU polymer used to form the drug loaded PEU film will have the formula: 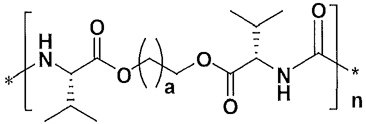 where a is an integer from 1 to 20; and n is an integer from 20 to 300. In various embodiments, the ranges for a and n may be as set forth above.

In some embodiments, the amino acid based PEU polymer may be synthesized using the two-step process shown in Scheme 1 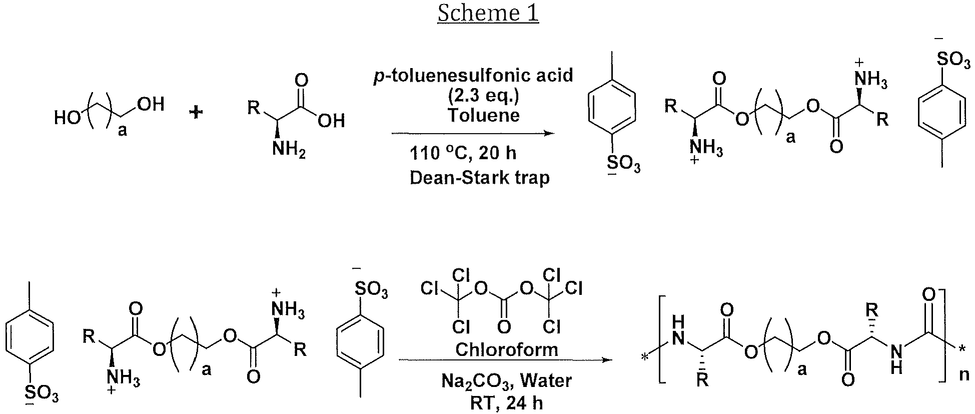 R = -CH₃, -(CH₂)₃NHC(NH₂)C=NH, -CH₂CONH₂,-CH₂COOH, -CH₂SH, -(CH₂)₂COOH, -(CH₂)₂CONH₂, -NH₂, - CH₂C=CH-N=CH-NH, -CH(CH₃)CH₂CH₃, -CH₂CH(CH₃)₂, -(CH₂)₄NH₂, -(CH₂)₂SCH₃, -CH₂Ph, -CH₂OH, - CH(OH)CH₃, -CH₂-C=CH-NH-Ph, -CH₂-Ph-OH,-CH(CH₃)₂, or -CH₂C₆H₄OCH₂C₆H₅; a = 1 - 20; n = 20 - 300.

In the embodiments shown in Scheme 1, the monomer is first synthesized *via* Fischer esterification between a linear C1-C20 diol and a selected amino acid that has been protonated using p-toluenesulfonic acid to prevent amidation and exchange reactions, as described above. For polymer synthesis, interfacial polymerization is then carried out using triphosgene to form urea linkages. In these embodiments, the polymer synthesis reaction further includes sodium carbonate, or another base, to deprotonate the amine groups of the monomer which is then free to react with the triphosgene. In various embodiments, the ranges for a and n may be as set forth above.

Next, the drug to be delivered is selected or synthesized as required for the desired application. The drugs that may be delivered using the drug-loaded degradable amino acid based poly(ester urea) film of the present invention are not particularly limited provided that they are not damaged, deactivated, and/or degraded by their interaction with the polymer or solvents used. In some embodiments, suitable drugs may include, without limitation, antibiotics, cancer drugs, antipsychotics, antidepressants, sleep aids, tranquillizers, anti-Parkinson's drugs, mood stabilizers, pain killers, anti-inflammatories, antimicrobials, and combinations thereof. In some embodiments, the drug to be delivered is an antibiotic. Suitable antibiotic may include, without limitation, lipopeptides, fluoroquinolone, lipoglycopeptides, cephalosporins, penicillins, monobactams, carbapenems, macrolide antibiotics, lincosamides, streptogramins, aminoglycoside antibiotics, quinolone antibiotics, sulfonamides, tetracycline antibiotics, chloraphenicol, metronidazole, tinidazole, nitrofurantoin, glycopeptides, oxazolidinones, rifamycins, polypeptides, tuberactinomycins, and combinations or pharmaceutically acceptable salts thereof. In some embodiments, the drug to be delivered may be cefazolin sodium or a pharmaceutically acceptable salt thereof.

As set forth above, the selected amino acid based PEU polymer and drug are then dissolved in a co-solvent to form the drug loaded PEU solution that will be used to form the drug loaded amino acid based PEU polymer solution used to form the films. The co-solvent for the drug and the degradable amino acid based poly(ester urea) polymer is not particularly limited and may be any suitable co-solvent for the selected amino acid based PEU polymer and drug at the relevant temperatures and concentrations, provided that the co-solvent does not cleave ester the bonds on the amino acid based poly(ester urea) polymer or damage, deactivate, and/or degrade the drug. In some embodiments, suitable co-solvents for the drug and the degradable amino acid based poly(ester urea) polymer may include, without limitation, polar solvents, methanol, ethanol, isopropanol, acetone, dioxane, DMF, THF, or combinations thereof.
In some embodiments, the co-solvent for the drug and the degradable amino acid based poly(ester urea) polymer is methanol.

The quantity co-solvent used must, of course, be enough to fully dissolve the selected amino acid based PEU polymer and drug, but should also be calculated to provide a viscosity suitable for the particular film forming method chosen.

As will be apparent to those of ordinary skill in the art, the overall drug loading for the drug-loaded degradable amino acid based poly(ester urea) films of the present invention is a function of the ratio of the degradable amino acid based poly(ester urea) polymer to the drug in the solution. In various embodiments, degradable amino acid based poly(ester urea) polymer to the drug in the solution from 100:1 to 1:1, preferably from 10:1 to 1:1; and more preferably from 5:1 to 1:1 by weight. In some embodiments, the ratio of the degradable amino acid based poly(ester urea) polymer to the drug in the solution may be from 80:1 to 1:1, in other embodiments, from 40:1 to 1;1, in other embodiments, from 20:1 to 1:1, in other embodiments, from 3:1 to 1:1, in other embodiments, from 100:1 to 50:1, in other embodiments, from 100:1 to 20:1, and in other embodiments, from 100:1 to 3:1. Here, as well as elsewhere in the specification and claims, individual range values can be combined to form additional non-disclosed ranges.

The drug-loaded degradable amino acid based poly(ester urea) film may be formed from the degradable amino acid based poly(ester urea) polymer / drug solution by any method known in the art for that purpose including, but not limited to, blade coating, solvent coating, lamination, spray coating, extrusion, injection molding, and combinations thereof. In some embodiments, the drug-loaded degradable amino acid based poly(ester urea) film of the present invention may be formed on a medical device by dip coating. In some embodiments, the drug-loaded degradable amino acid based poly(ester urea) film of the present invention is formed by blade coating.

In some of these embodiments, the drug-loaded degradable amino acid based poly(ester urea) film is made using standard blade coating techniques as shown in FIGS. 1A-F. As set forth above, the drug loaded amino acid based PEU solution (PEU-A solution) is first formed by dissolving the amino acid based PEU polymer and the drug to be delivered in a co-solvent as described above and shown in FIG. 1A. A representative blade coating apparatus 10 for use in preparing the film in these embodiments is shown in FIG. 1B. The blade coating apparatus 10 comprising a base 12 having an upper surface 14, on which is placed a substrate 16 upon which the film will be cast. Blade coating apparatus 10 further comprises casting blade box 18, which is located on substrate 16 (if present) or base 12, and is free to slide across substrate 16 (if present) or base 12. The choice of substrate in these embodiments, is not particularly limited. As will be appreciated, if the film is to be removed from the substrate, the selected substrate should not form co-valent bonds with the polymer. Suitable substrates may include, without limitation, substrates comprising poly(ethylene terephthalate) (PET), polytetrafluoroethene (PTFE) (TEFLON^{™}), poly(4,4'-oxydiphenylene-pyromellitimide) (KAPTON^{™}), polyethylene, polypropylene, or a combination thereof. In some embodiments, the substrate may comprise the implantable medical device itself, in which case, base 12 may or may not be omitted depending upon the size and nature of the device upon which the film is to be formed.

In these embodiments, the casting blade box 18 will contain an adjustable doctor blade 20, having a bottom end 22, defining a gap 24 between the bottom end 22 of the adjustable doctor blade 20 and an upper surface 26 of substrate 16 (if present) or the upper surface 14 of base 12. (See FIGS. 1B-E) Casting blade box 18 further comprises solution well 28. As can be seen in FIGS. 1C-E, solution well 28 holds the drug loaded amino acid based PEU solution (PEU-A solution) 29 and is in fluid communication with gap 24. As the casting blade box 18 is slid backwards (away from gap 24) the PEU-A solution forms the drug loaded PEU film 30 having an initial thickness equal to the height of gap 24. (See FIG. 1C) Adjustable doctor blade 20 may be adjusted up and down using adjustment screws 32 to change the height of the gap 24, and with it the initial thickness of the drug loaded PEU film 30. Moving the adjustable doctor blade up will increase the initial thickness of the drug loaded PEU film being formed (See FIG. 1D) and moving the adjustable doctor blade down will decrease the initial thickness of film 24 being formed by the blade coating apparatus 10. (See FIG. 1E)

After the film dries, it may be removed from the substrate as shown in FIG. FIG. 1F. As will be appreciated, while the initial thickness of the drug loaded PEU film may be controlled by height of gap 24 as set forth above, the thickness of the film will decrease as it dries, and the amount of that decrease will depend upon the particular characteristics (i.e. the amount/concentration of solvent, the molecular weight of the polymer, the drug loading etc.) of the amino acid based poly(ester urea) polymer and drug solution being used. This fact should, of course, be considered when determining what thickness of film should be formed on the substrate in order to produce a film having a predetermined thickness. Also, as noted above, it is believed that the drug is substantially evenly distributed throughout the film, most likely because the film is formed while the drug is solution with the polymer, and is trapped in the polymer as the solvent evaporates and the polymer solidifies.

In some embodiments, blade coating apparatus is adjusted to form films having a thickness of from 1µm to 100 µm, preferably from 2 µm to 500 µm, and more preferably from 10 µm to 150 µm. In some embodiments, the blade coating apparatus is adjusted to form films having a thickness of from 40 µm to 140 µm.

### Experimental

To further evaluate the antibiotic-loaded poly(ester urea) films (PEU-A film) of the present invention as a candidate for fabricating envelope to cover CIED during implantation and further reduce it to practice, a series of antibiotic-loaded amino acid based poly(ester urea) films were formed and characterized. Unlike other poly(α-amino acids), there PEU polymers have tunable physical, mechanical and degradation properties. The byproducts of PEU degradation are amino acids, carbon dioxide, and diacids, all of which can be resorbed and excreted naturally. PEUs have shown a limited inflammatory response in previous studies *in vivo.* The L-Valine based PEU allow for attachment and spreading of cell lines that are relevant to smooth muscles and soft tissues. In these experiments, L-valine based poly(ester urea) p(1-VAL-8) was synthesized and characterized. The polymer p(1-Val-8) PEU was then used to fabricate a series of antibiotic-loaded films with varying antibiotic loading concentration (2%, 5%, 10%) and thickness (40 µm, 80 µm, 140 µm). These films were analyzed for *in vitro* antibiotic release over a period of 14 days. To assess the effectiveness of these films in bacterial clearance, antimicrobial testing was also done.

**Synthesis.** The monomer was synthesized *via* Fischer esterification between 1,8 octane diol and L-valine amino acid which was protonated using p-toluenesulfonic acid to prevent amidation and exchange reactions (Scheme 2). 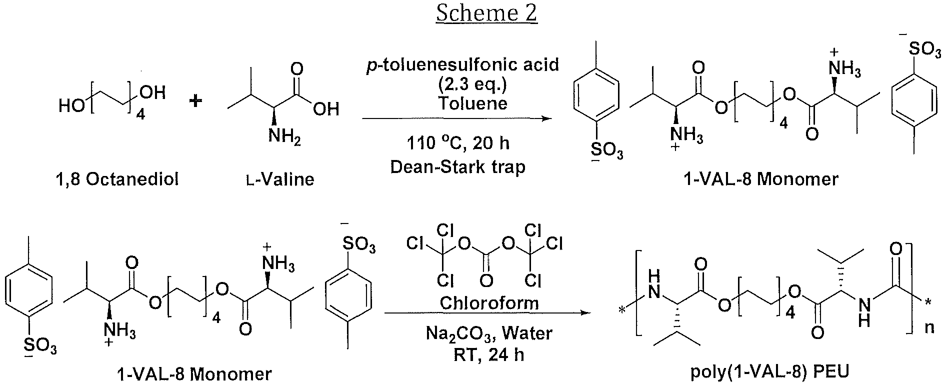 As can be seen in Scheme 2, the amino acid-based PEU polymer was synthesized by interfacial polymerization using triphosgene to form urea linkages and sodium carbonate to deprotonate the amine groups of monomer which then further reacted with triphosgene.

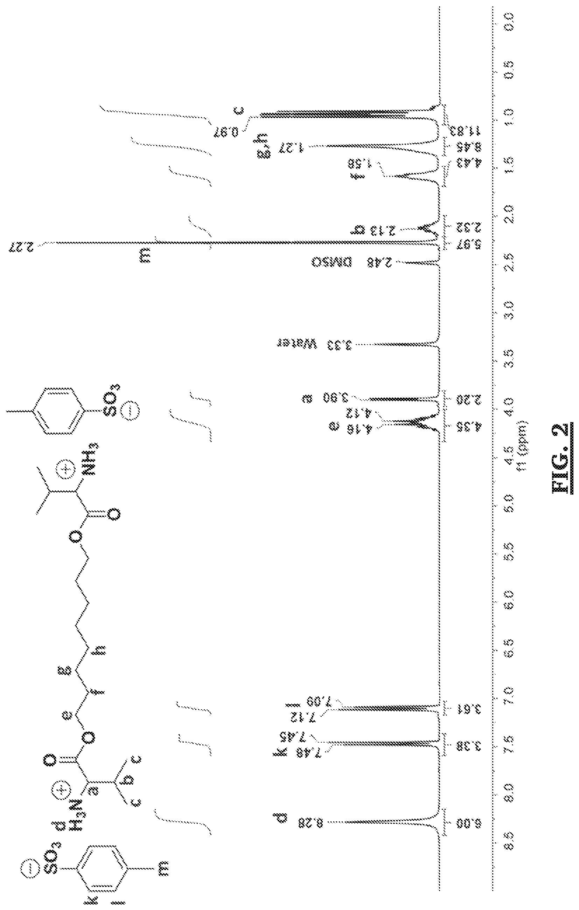

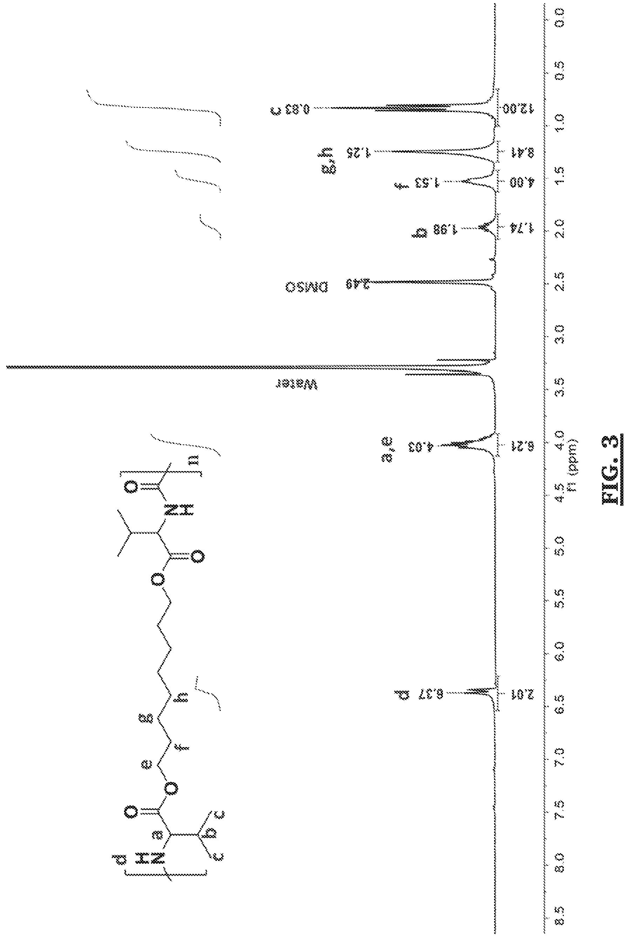

The synthesis was confirmed by ¹H-NMR (FIG. 2). For polymer synthesis, interfacial polymerization was carried out using triphosgene to form urea linkages. The sodium carbonate was used to deprotonate the amine groups which further reacted with triphosgene to form the p(1-VAL-8) PEU (Scheme 2). The synthesis was confirmed by ¹H-NMR and ATR-FTIR (FIGS. 2, 3). The L-valine amino acid was chosen for its less rigid side chain when compared to previously studied L-phenylalanine and L-tyrosine, which affords greater chain flexibility. Since the polymer is extracted by precipitation, some of the lower molecular mass chains are fractionated resulting in *Ð*ₘ less than the theoretical value of 2.0.

**Fabrication.** Doctor blade assisted solution casting was used to fabricate the PEU-antibiotic (PEU-A) films. A total of 9 groups with varying thickness (40 µm, 80 µm, 120 µm) and antibiotic loading (2 wt%, 5 wt%, 10 wt%) were fabricated. Table 1, below enumerates the groups, their thickness, antibiotic loading, and actual antibiotic loading content as determined by content uniformity testing. The groups in Table 1 are named based on their antibiotic loading concentration and thickness. For example, the group "2%-40µm" corresponds to PEU-antibiotic (PEU-A) film with 2 wt% cefazolin and 40 µm thickness.

Blade assisted solution casting is advantageous as it allows for the fabrication of PEU-A film with a desired thickness and loading concentration reproducibly, without having to manipulate several parameters. The thickness can be adjusted by merely changing the gap height and the loading concentration of the film can be adjusted by changing the concentration of PEU-A solution. It can be easily scaled up to a Roll-to-roll casting line. Unlike extrusion or additive manufacturing, it avoids thermal processing and UV exposure which could degrade the incorporated antibiotic and reduce its bioactivity. Additionally, during fabrication of drug delivery systems, many API tend to undergo polymer matrix induced crystallization. The crystalline state of an API is thermodynamically more stable than the amorphous state. Its solubility and dissolution rate are lower than that of the amorphous phase this could potentially have a negative impact on bioavailability. Results from antibacterial assays suggest the feasibility of blade assisted solution casting for fabrication of PEU-A films as antibiotic released from PEU-A retains its activity and is bioavailable to eliminate bacteria.

**Table 1**

| **Groups** | **Thickness (µm)** | **Theoretical antibiotic concentration** | **Actual antibiotic content*** |
|---|---|---|---|
| **2%-40µm** | 40 ± 5 | 2% | 1.98 ± 0.13 % |
| **2%-80µm** | 80 ± 10 | 2% | 1.97 ± 0.08 % |
| **2%-140µm** | 140 ± 10 | 2% | 1.98 ± 0.18 % |
| **5%-40µm** | 40 ± 5 | 5% | 4.83 ± 0.34 % |
| **5%-80µm** | *80* ± *10* | *5%* | *4.81* ± *0.19 %* |
| **5%-140µm** | 140 ± 10 | 5% | 4.86 ± 0.44 % |
| **10%-40µm** | 40 ± 5 | 10% | 9.47 ± 0.15 % |
| **10%-80µm** | 80 ± 10 | 10% | 9.58 ± 0.37 % |
| **10%140µm** | 140 ± 15 | 10% | 9.49 ± 0.26 % |

| | | | |
|---|---|---|---|
| *The actual antibiotic content was calculated from content uniformity results. | | | |

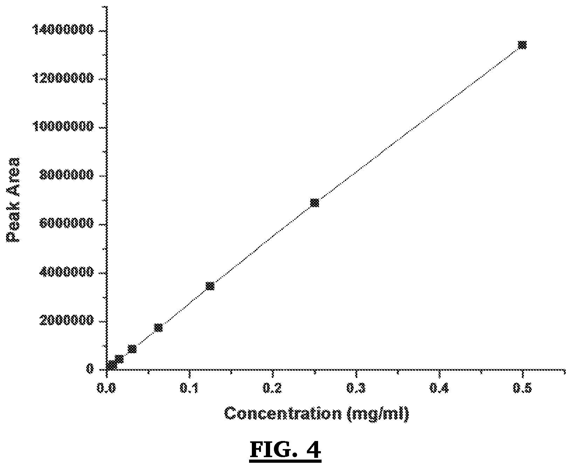

***In vitro* antibiotic release.** *In* vitro release profile can reveal fundamental information on the dosage form and its behavior, as well as provide details on the release mechanism and kinetics. This enables a rational and scientific approach to drug product development. The antibiotic release profiles of PEU-A films were collected over a period of 14 days. To determine the drug release of PEU-A films a 12mm discs were punched out from each film and kept under sink conditions. The amount antibiotic of release was calculated from sink concentrations, which were determined from HPLC analysis and compared to a calibration curve (FIG. 4). Samples were placed in PBS (3 mL) at 37 °C, and the sink was replaced at each timepoint. (n = 5 samples per group) FIGS. 5D-F show the cumulative release profile for all 9 groups of PEU-A films, while FIGS. 5A-C show the cumulative percent release profile for corresponding groups. The films are grouped based on their thickness. The dot and dash line in cumulative release profiles indicates a label claim for each PEU-A group. The label claim for PEU-A films was determined from content uniformity testing. The percentage of cumulative antibiotic released at each timepoint was calculated based on label claim. Label claim 100% of antibiotic in a PEU disk which was determined from content uniformity testing. Content uniformity helps to quantify actual antibiotic content in the film and helps to characterize whether the antibiotic is uniformly dispersed over the surface entire area of the film.

Several models were used to fit the release data. The release profile and conditions of release fit well in the Higuchi model and fulfill all of its assumptions. (See, Table 2, below.

**Table 2**

| *The assumption of the Higuchi model and explanation on how the experiment meets the requirements* | |
|---|---|
| **Higuchi Model Assumptions** | **Explanation on how the experiment meets the requirements** |
| 1. The sample is placed in a "perfect sink." | *The volume of the sink at each timepoint was at least 5 times greater than the volume required to solubilize entire amount of antibiotic (Cefazolin) present in the film.* |
| 2. The initial drug concentration in the film is much higher than the solubility of the drug in the polymer. | *The polymer p(1-VAL-8) is an amorphous solid and does not solubilize Cefazolin.* |
| 3. The drug is finely dispersed within the polymer matrix. | *This was confirmed through content uniformity testing* |
| 4. The dissolution of drug particles is rapid compared to the diffusion of dissolved drug molecules. | *Cefazolin has high solubility in water (> 100mg*/*ml) and its diffusion through the film is the rate-limiting step.* |
| 5. The surface area of the film is large compared to its thickness. | *The sample discs for release study have 12* mm *(12000 µm) diameter which is much higher as compared to PEU-A films which have a thickness of 40 µm, 80 µm or 140 µm*. |
| 6. The polymer does not swell or dissolve during drug release. | *The polymer is water-insoluble and does not undergo significant hydrolytic degradation in the time frame for this experiment.* |

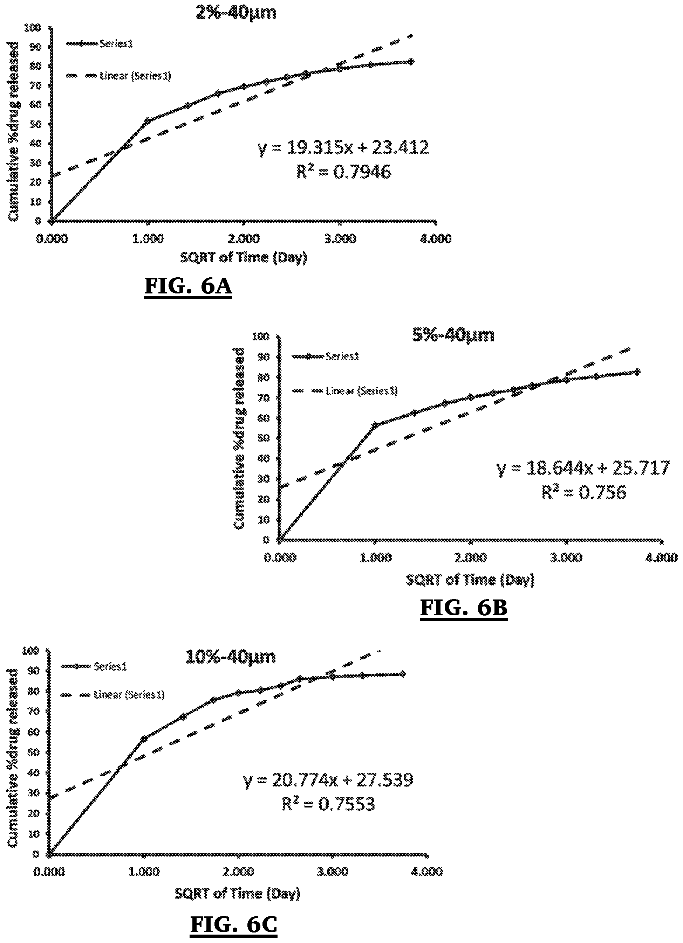

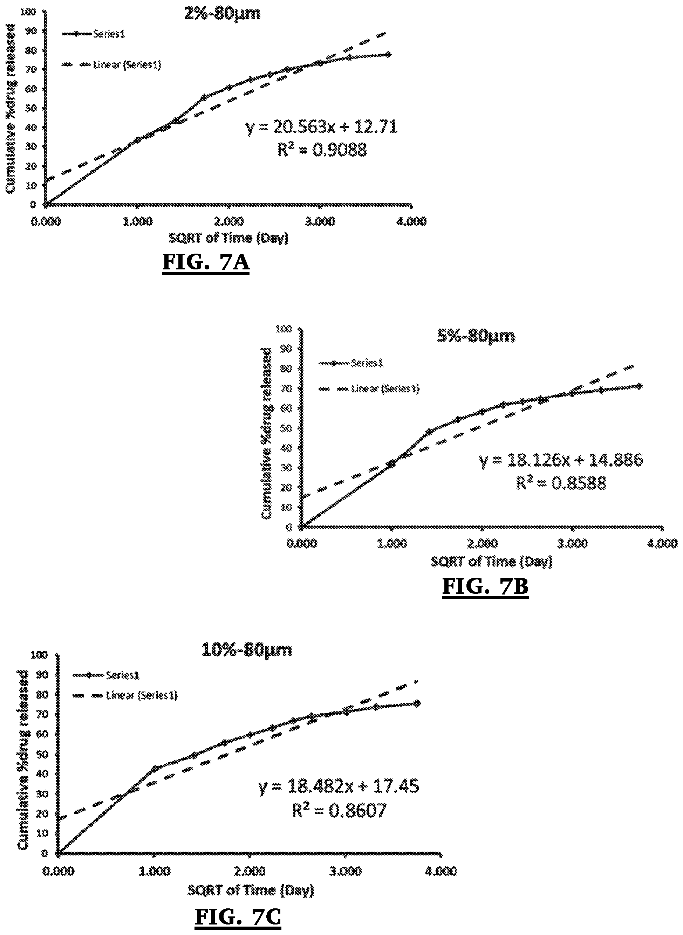

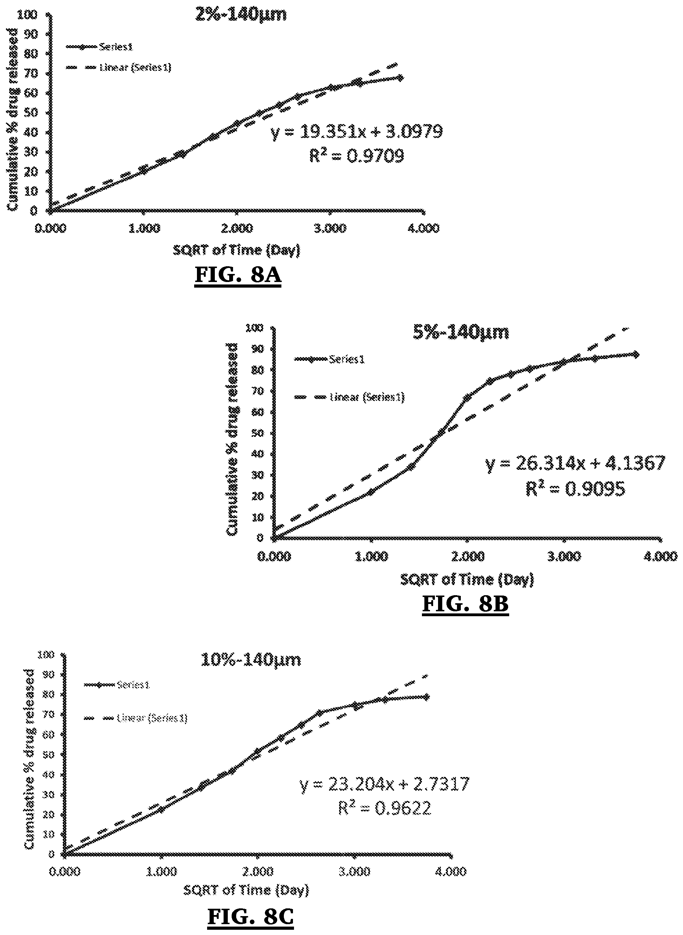

The fitting graphs are shown in FIGS. 6A-C, 7A-C, and 8A-C. The Higuchi model implies that antibiotic diffusion through the PEU matrix is diffusion controlled. The Higuchi model can be used to explain the release profiles for PEU-A films, as to why groups with higher thickness have slower cumulative percent release. This is because with time the diffusion front moves inwards form the edges across its cross-section. Thus, for films with higher thickness, the diffusion front will travel lower percentage of film's cross-section as compared to lower thickness film in the same amount of time resulting in slower percent cumulative release. FIGS. 9A-B indicates that for groups with the same thickness, the amount of antibiotic released varies based on its loading. This is because even though the diffusion front moves at the same rate through the films with the same thickness, the amount of antibiotic dispersed across cross-section changes based on its loading concentration. Thus, over a period of time groups with higher loading show higher antibiotic release. It could be hypothesized that higher thickness would lead to sustained release over a longer period.

It has previously been shown that delivering antibiotic locally for 7-10 days at the implant site can reduce CIED infection cases by 80 %. Thus, the aim was to develop a drug delivery matrix that can deliver a therapeutically relevant amount of antibiotic locally for 2 weeks.

As will be appreciated by those of skill in the art, initial burst release is a common phenomenon in most polymer-based delivery systems which severely affects their ability for sustained drug release. This is especially challenging to control in film-based delivery systems. It is worth noting that groups, 2%-140µm, 5%-140µm, 10%-140µm have a modest percent antibiotic release of 20.2 %, 21.8%, 22.5% in first 24 hours respectively. PEU-A films show the ability to delivery antibiotic for 12-14 days. Moreover, each film group has a distinct release profile which varies based on its thickness and antibiotic loading concentration. Thus, thickness and loading concentration influence the amount and rate of antibiotic release. This dependence of release on thickness and loading concentration give a handle to fabricate PEU-A films with any desired release profile that can deliver the therapeutically relevant amount of antibiotic for about 2 weeks. This property can be employed to design dosage specific films that could cater to the needs of patients in different age groups and suffering from diverse medical conditions.

**Antimicrobial Testing.** To assess whether the antibiotic release through PEU-A films retains its activity antimicrobial assays were performed. A blank PEU film without any antibiotic was fabricated to act as a control in these assays. Zone inhibition assay on blank PEU indicates that PEUs do not possess inherent antimicrobial properties (FIGS. 9A-B). Both the disc diffusion and well diffusion assays were performed using an agar plate, setup shown in FIGS. 10A-B. The activity of the antibiotic released from each group was determined by measuring the diameter of the inhibition zone (IZ) around the sample. The diameter of IZ depends on the antimicrobial activity of antibiotic, diffusivity of antibiotic through agar and minimum inhibitory concentration (MIC) of a specific pathogen. Thus, a group that releases a higher amount of antibiotic has bigger IZ. Accordingly for disc diffusion assay, 10% groups (10%-40µm, 10%-80µm, 10%-140µm) have greater IZ than 5% groups(5%-40µm, 5%-80µm, 5%-140µm), which in turn have higher IZ than 2% (2%-40µm, 2%-80µm, 2%-140µm) groups. A similar trend is also observed for well diffusion assay, with bigger IZ. (See, FIGS. 9A-B) and addition of broth to the well in agar helps further diffusion of antibiotic resulting in bigger IZ.

For the broth incubation assay, the sample discs were incubated with growth broth and bacterial inoculum for 24 h in a microtiter plate. Well with PEU-A discs had significantly lower Colony forming units (CFU) when compared to negative controls. Thus, all sample groups inhibited bacterial growth in the broth. The extent of bacterial inhibition depended on the amount of antibiotic released. Overall, results from antimicrobial susceptibility experiments indicate that antibiotic released from samples retained its antimicrobial activity. (See, FIG. 11)Thus, the current processing method for fabricating films is suitable for the antibiotic as it retains its antimicrobial activity after film fabrication and is bio-available once released. PEU- A films prove to be effective in bacterial clearance, thus eliminating infection-causing bacteria in its initial stages. This could prevent damage to CIED and surrounding tissue.

It should be noted that cefazolin was used as a model antibiotic for this study. Antibiotics with higher antibacterial activity and spectrum are available which could prove even more effective in preventing CIED infections. Our preliminary studies indicate that picking appropriate solvent for solution casting is crucial as it dictates homogeneity of PEU-A solution. Inhomogeneous solutions result in films with uneven distribution of antibiotic. Owing to the solubility of PEUs in several solvents (e.g. Methanol, Ethanol, THF, Acetone, IPA etc.) other antibiotics or a combination of antibiotics could be employed to fabricate the PEU-A films. Our results suggest the feasibility of PEU-A films as an effective long-term sustained release matrix for localized delivery of antibiotics.

As set forth above, despite improvements in prophylactic practices the occurrence of CIED infections has not declined. The conventional, systemic antibiotic delivery is less efficacious in many cases and therefore localized antibiotic delivery must be employed in a supplement to systemic administration. Thus, an envelope or pouch covering CIED that can locally deliver the antibiotic in tissue pocket can be effective in peri-operative bacterial clearance preventing severe chronic infection and the need for replacing CIED. Herein we investigated antibiotic-loaded poly(ester urea) film (PEU-A film) as a candidate for making an envelope to cover CIED during implantation. The polymer p(1-Val-8) PEU was used to fabricate a series of antibiotic-loaded films with varying antibiotic loading concentration (2%, 5%, 10%) and thickness (40 µm, 80 µm, 140 µm). *In vitro* antibiotic release shows that each film group had a distinct release profile. Thickness and loading concentration influence the amount and rate of antibiotic release. This dependence of release on thickness and loading concentration gives a handle to fabricate PEU-A films with any desired release profile, which can deliver the therapeutically relevant amount of antibiotic. Group 10%-140µm showed a sustained release of antibiotic for 14 days and was found most effective in bacterial clearance. Since PEUs are soluble in several solvents other antibiotics or a combination of antibiotics could be effortlessly employed to fabricate the PEU-A films. Our results suggest the feasibility of PEU-A films as an effective long-term sustained release matrix for localized delivery of antibiotics.

### EXAMPLES

The following examples are offered to more fully illustrate the invention, but are not to be construed as limiting the scope thereof. Further, while some of examples may include conclusions about the way the invention may function, the inventor do not intend to be bound by those conclusions, but put them forth only as possible explanations. Moreover, unless noted by use of past tense, presentation of an example does not imply that an experiment or procedure was, or was not, conducted, or that results were, or were not actually obtained. Efforts have been made to ensure accuracy with respect to numbers used (e.g., amounts, temperature), but some experimental errors and deviations may be present. Unless indicated otherwise, parts are parts by weight, molecular weight is weight average molecular weight, temperature is in degrees Centigrade, and pressure is at or near atmospheric.

### Materials.

1,8-Octanediol, L-valine, sodium carbonate, activated carbon black, triphosgene, p-toluenesulfonic acid monohydrate, toluene, chloroform, methanol, monobasic sodium phosphate monohydrate, phosphoric acid, DMF, acetonitrile (HPLC grade), water (HPLC grade) were purchased from Sigma-Aldrich or Fisher Scientific. Cefazolin was purchased from TCI America. *S. aureus* (ATCC 25923) was acquired from ATCC (Manassas, VA). All solvents and chemicals were used without further purification.

### Analytical Methods and Equipment

¹H NMR spectra were collected using a 300 MHz Varian NMR spectrophotometer. Chemical shifts are reported in ppm (δ) and referenced to residual solvent resonances (¹H NMR, DMSO-*d*₆: 2.50 ppm). Multiplicities were explained using the following abbreviations: s = singlet, d = doublet, t = triplet, and m = multiplet. Size exclusion chromatography (SEC) was performed using an EcoSEC HLC-8320GPC (Tosoh Bioscience, LLC) equipped with a TSKgel SuperH-RC 6.0 mm I.D. × 15 cm mixed bed column and refractive index (RI) detector. The number average molecular mass (*M*ₙ), weight average molecular mass (*M*_{w}), and molecular mass distribution (*Ð*_{M}) for each sample was calculated using a calibration curve determined from poly(styrene) standards (PStQuick MP-M standards, Tosoh Bioscience LLC) with 0.01 M LiBr in DMF as the eluent flowing 1.0 mL/min at 50 °C. Differential scanning calorimetry (DSC) was performed using a TA Q200 scanning a temperature range from -10°C to 100 °C with heating and cooling rates of 10 °C/min. The glass transition temperature (*T*_{g}) was determined from the midpoint of the transition in the second heating cycle. Thermogravimetric analysis (TGA) was performed using a TA Q500 with heating ramps of 10 °C/min in the temperature range from 0 to 700 °C. The degradation temperature (*T*_{d}) was determined from 5% mass loss. Attenuated total reflection-Fourier transform infrared spectroscopy (ATR-FTIR) was performed on an Excalibur FTS 3000 ATR-FTIR. The wavenumber (cm⁻¹) range for the ATR-FTIR spectra was 4000-500 cm⁻¹.

### Example 1

### Synthesis of di-p-toluenesulfonic acid salts of bis(L-valine)-octane 1,8-diester monomer (1-VAL-8).

Synthesis of 1-VAL-8 monomer was carried out following previously published procedures (See, Scheme 2, above). Briefly, 1,8-octanediol (40 g, 0.27 mol, 1 equiv), L-valine (72.6 g, 0.62 mol, 2.3 equiv), p-toluenesulfonic acid monohydrate (128.3 g, 0.68 mol, 2.5 equiv), and toluene (900 mL) were added to a 2 L round-bottom flask and mixed using magnetic stirrer. A Dean-Stark Trap was attached to the round-bottom flask, and the reaction was heated to reflux for 24 h. The reaction was cooled to ambient temperature, and the resulting white precipitate was isolated by vacuum filtration using a Buchner funnel. The product was recrystallized by dissolving in boiling water (2 L), vacuum filtering hot, and cooling to room temperature to obtain a white solid precipitate. The precipitate was collected via filtration and the process was repeated 3 times to maximize purity (81% yield).

**(1-VAL-8) monomer** ¹H-NMR (300 MHz, DMSO-*d*₆, 30 °C) δ ppm 0.97 (m, 12H) 1.27 (s, 8H) 1.58 (m, 4H) 2.13 (m, 2H) 2.27 (s, 6H), 3.90 (d, 2H) 4.12-4.16 (m, 4H) 7.09-7.12 (d, 4H) 7.45-48 (d, 4H) 8.28 (s, 6H).

### Example 2

### Synthesis of Di-p-toluenesulfonic Acid Salts of Bis(L-valine)-Octane 1,8-Diester Monomer (1-VAL-8).

Synthesis of di-p-toluenesulfonic acid salts of bis(*L*-valine)-octane 1,8-diester (1-VAL-8) was carried out following previously published procedures. Briefly, in a 1 L 1-neck round bottom flask, 1,8-octanediol (43.8 g, 0.3 mol, 1 eq.), *L*-valine (73.8 g, 0.63 mol, 2.3 eq.), *p*-toluenesulfonic acid monohydrate (131.3 g, 0.69 mol, 2.4 eq.), and toluene (1300 mL) were added and equipped with a stir bar. A Dean-Stark trap was fastened to the round bottom flask and the reaction was heated to 110 °C and allowed to reflux for 24 h. The reaction was cooled to room temperature, and the resulting white precipitate was isolated by vacuum filtration using a Buchner funnel. The product was dissolved in boiling water (1 L), hot vacuum filtered, and cooled to room temperature to further purify the white solid precipitate. The precipitate was collected via filtration and the recrystallization process was performed three times for purity (82% yield). The resulting product was characterized by Proton Nuclear Magnetic Resonance Imagery (¹H NMR) ((300 MHz, 303 K, DMSO-*d₆*): *δ =* 0.94 (m, 12H), 1.28 (s, 8H), 1.59 (m, 4H), 2.07-2.18 (m, 2H), 2.27 (s, 6H), 2.50 (m, DMSO), 3.33-3.38 (s, H₂O), 3.89 (d, ³*J*_{H}-_{H} = 3.0 Hz, 2H), 4.07-4.23 (m, 4H), 7.07-7.23 (d, ³*J*_{H}-_{H} = 8.2 Hz, 4H, aromatic H ), 7.45-7.48 (d, ³*J*_{H}-_{H} = 8.1 Hz, 4H, aromatic H), 8.25 (br, 6H) ppm).

### Example 3

### Synthesis of Di-p-toluenesulfonic Acid Salts of Bis(L-valine)-Octane 1,8-Diester Monomer (1-VAL-8).

Synthesis of di-*p*-toluenesulfonic acid salts of bis(*L*-valine)-octane 1,8-diester (1-VAL-8) was carried out following previously published procedures. 1,8-octanediol (43.8 g, 0.3 mol, 1 eq.), *L*-valine (73.8 g, 0.63 mol, 2.3 eq.), p-toluenesulfonic acid monohydrate (131.3 g, 0.69 mol, 2.4 eq.), and toluene (1300 mL) were added to a 3 L 3-neck round bottom flask and mixed with overhead mechanical stirring. A Dean-Stark Trap was attached to the round bottom flask and the reaction was heated to reflux for 24 h. The reaction was cooled to ambient temperature, and the resulting white precipitate was isolated by vacuum filtration using a Buchner funnel. The product was recrystallized by dissolving in boiling water (2 L), vacuum filtering hot, and cooling to room temperature to afford a white solid precipitate. The precipitate was collected via filtration and the recrystallization process was performed three times for purity (166 g, 79% yield). The resulting product was characterized by ¹H NMR (300 MHz, DMSO-*d₆*): *δ* = 0.95-0.99 (m, 12H, -CH(C**H₃**)₂), 1.24-1.35 (s, 8H, -COOCH₂CH₂(C**H₂**)₄-), 1.55-1.65 (m, 4H, -COOCH₂C**H₂**(CH₂)₄C**H**₂-), 2.06-2.22 (m, 2H, (CH₃)₂C**H**-), 2.26-2.31 (s, 6H, -CH₃Ar-), 2.50 (m, DMSO), 3.33-3.38 (s, H₂O), 3.88-3.90 (d, *J* = 4.3 Hz, 2H, ⁺NH₃C**H**COO-), 4.08-4.24 (m, 4H, - COOC**H₂**CH₂(CH₂)₄-), 7.10-7.14 (d, *J* = 8.2 Hz, 4H, aromatic H ), 7.48-7.50 (d, *J* = 8.1 Hz, 4H, aromatic H), 8.25-8.33 (br, 6H, -NH₃⁺).

### Example 4

### Synthesis of Di-p-toluenesulfonic Acid Salts of Bis(L-valine)-Decane 1,10-Diester Monomer. (1-VAL-10).

Synthesis of di-*p*-toluenesulfonic acid salts of bis(*L*-valine)-decane 1,10-diester (1-VAL-10) was carried out using the method shown in Example 3, above with 1,10 decanediol as the diol (154 g, 71% yield). The resulting product was characterized by ¹H NMR ((300 MHz, DMSO-*d₆*): *δ* = 0.93-1.00 (m, 12H, -CH(C**H₃**)₂-), 1.22-1.33 (s, 12H, - COOCH₂CH₂(C**H₂**)₆-), 1.55-1.64 (m, 4H, -COOCH₂C**H₂**(CH₂)₄C**H₂**-), 2.09-2.21 (m, 2H, (CH₃)₂C**H**-), 2.28-2.31 (s, 6H, -CH₃Ar-), 2.50 (m, DMSO), 3.30-3.35 (s, H₂O), 3.87-3.91 (d, *J* = 4.5 Hz, 2H, ⁺NH₃C**H**COO-), 4.08-4.24 (m, 4H,-COOC**H₂**CH₂(CH₂)₆-), 7.10-7.13 (d, *J* = 7.8 Hz, 4H, aromatic H), 7.47-7.51 (d, *J* = 7.8 Hz, 4H, aromatic H), 8.27-8.31 (br, 6H, -NH₃⁺)).

### Example 5

### Synthesis of Di-p-toluenesulfonic Acid Salts of Bis-(l-valine)-Dodecane 1,12-Diester Monomer. (1-VAL-12).

Synthesis of di-*p*-toluenesulfonic acid salts of bis(*L*-valine)-dodecane 1,12-diester (1-VAL-12) was carried out using the method shown in Example 3, above with 1,12 dodecanediol as the diol (106 g, 82% yield). The resulting product was characterized by ¹H NMR (300 MHz, DMSO-*d₆*): *δ* = 0.90-0.98 (m, 12H,-CH(C**H₃**)₂), 1.22-1.27 (s, 16H, - COOCH₂CH₂(C**H₂**)₈-), 1.53-1.63 (m, 4H, -COOCH₂C**H₂**(CH₂)₈C**H₂**-), 2.07-2.18 (m, 2H, (CH₃)₂C**H**⁺-), 2.27-2.29 (s, 6H, -CH₃Ar-), 2.50 (m, DMSO), 3.29-3.33 (s, H₂O), 3.87-3.90 (d, *J* = 4.3 Hz, 2H, ⁺NH₃C**H**COO-), 4.06-4.22 (m, 4H, -COOC**H₂**CH₂(CH₂)₈-), 7.08-7.11 (d, *J* = 7.9 Hz, 4H, aromatic H), 7.45-7.49 (d, *J* = 8.1 Hz, 4H, aromatic H), 8.25-8.28 (br, 6H, -NH₃⁺)).

### Example 6

### Synthesis of Di-p-toluene Sulfonic Acid Salts of Bis-L-phenylalanine-hexane-1,6-diester Monomer. (1-PHE-6)

*Di-p-toluene* sulfonic acid salts of bis-*L*-phenylalanine-diol-diester monomers were prepared using previously published procedures, (See, Yu, J.; Lin, F.; Lin, P.; Gao, Y.; Becker, M. L. Macromolecules 2014, 47, 121; Lin, F.; Yu, J.; Tang, W.; Zheng, J.; Xie, S.; Becker, M. L. Macromolecules 2013, 46, 9515; and Pang, X.; Chu, C.-C. Biomaterials 2010, 31, 3745), and as shown in Scheme 3, below.

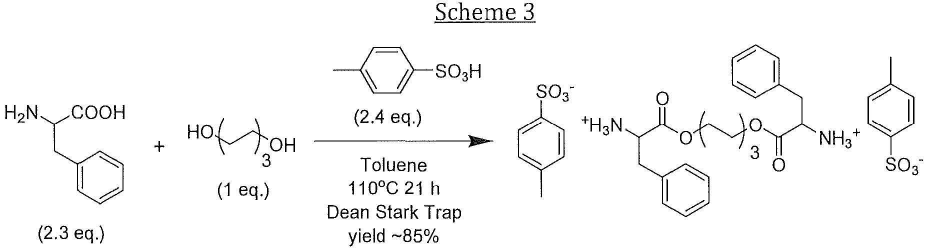

In brief, 1,6-hexanediol (10.0g, 90mmol, 1.0 equiv.), *L*-phenylalanine (32.2g, 200mmol, 2.3 equiv.), *p*-toluene sulfonic acid monohydrate (TsOH) (38.7g, 200mmol, 2.4 equiv.), and toluene (200mL) were mixed in a 500mL 2-neck round bottom flask equipped with Dean-Stark trap and a magnetic stir bar. The TsOH acidifies the solution conditions preventing the amidation of the carboxylic acids. The Dean Stark trap was used to collect the water biproducts, increasing the yield of the reaction. The system was heated to reflux (110°C) and purged with nitrogen for 20h. After 20h, the reaction mixture was cooled to ambient temperature and the product was filtered with diethyl ether. The solid product was dissolved in 3L of hot water and decolored using activated carbon black (2.0g) for 2-3 minutes and the decolorized hot liquid obtained by vacuum filtration. When the decolorized hot liquid was cooled to room temperature, the white solid product formed was collected by vacuum filtration. The white solid product was recrystallized 3 times using 3L of water to yield 57.0g (yield 85%) of the di-*p*-toluene sulfonic acid salt of bis-*L*-phenylalanine-1, 6-hexanediol-diester as a white powder. The compound produced was characterized by ¹H NMR ((300MHz, DMSO-d₆): 1.04-1.13 (m, 4H, -COOCH₂CH₂C***H***₂-) 1.38-1.44 (m, 4H, -COOCH₂C***H***₂CH₂-) 2.27 (s, 6H, C**H**₃Ar-) 2.50 (DMSO) 2.98-3.19 (m, 4H, -CHC***H***₂-Ar-) 3.89-4.03 (m, 4H, -COOC***H***₂CH₂-) 4.25-4.32 (m, 2H, ⁺NH₃C***H***COO-) 7.09-7.13 (d, 4 H, aromatic H) 7.21-7.34 (m, 10H, aromatic H) 7.47-7.50 (d, 4H, aromatic H) 8.36 (s, 6H, ⁺N***H***₃-)) and by ¹³C-NMR ((75 MHz, DMSO-*d₆*): 20.75, 24.66, 27.62, 35.97, 38.80-40.28 (DMSO-d₆), 53.07, 65.46, 125.39, 127.14, 127.95, 128.49, 129.30, 134.69, 137.78, 145.33, 169.03).

### Example 7

### Synthesis of Di-p-toluenesulfonic Acid Salts of Bis(_{L}-phenylalanine)-Hexane 1,6-Diester Monomer. (1-PHE-6).

Synthesis of di-*p*-toluene sulfonic acid of bis(*L*-phenylalanine)-hexane 1,6-diester (1-PHE-6) was carried out following previously published procedures. Briefly, in a 3 L 3-neck round bottom flask, 1,6-hexanediol (1 eq.), *L*-phenylalanine (2.3 eq.), *p-*toluenesulfonic acid monohydrate (2.4 eq.), and toluene (1300 mL) were added to a 1 neck flask and equipped with a stir bar. A Dean-Stark trap was fastened to the round bottom flask and the reaction was heated to 110 °C and allowed to reflux for 24 h. The reaction was cooled to room temperature, and the resulting white precipitate was isolated by vacuum filtration using a Buchner funnel. The product was dissolved in boiling water (2 L), hot vacuum filtered, and cooled to room temperature to further purify the white solid precipitate. The precipitate was collected via filtration and the recrystallization process was performed three times for purity. (81% yield). The compound produced was characterized by ¹H NMR ((300 MHz, 303 K, DMSO-*d₆*): *δ* =1.06 (s, 4H), 1.38 (m, 4H), 2.27 (s, 6H), 2.50 (m, DMSO), 2.96-3.17 (m, 4H), 4.01 (t, ³*J*_{H}-_{H} = 9.0 Hz, 4H), 4.28 (t, ³*J*_{H}-_{H}=6.0 Hz, 2H), 7.08-7.11 (d, 4 H), 7.20-7.35 (m, 10H), 7.45-7.48 (d, 4H), 8.37 (s, 6H) ppm).

### Example 8

### Synthesis of Di-p-toluenesulfonic Acid Salts of Bis(l-phenylalanine)-Hexane 1,6-Diester Monomer. (1-PHE-6).

Synthesis of di-p-toluene sulfonic acid of bis(*L*-phenylalanine)-hexane 1,6-diester (1-PHE-6) was carried out using the method described in Example 7, above (73% yield). The compound produced was characterized by ¹H NMR ((300 MHz, 303 K, DMSO-*d₆*): *δ* =1.06 (s, 4H), 1.38 (m, 4H), 2.27 (s, 6H), 2.50 (m, DMSO), 2.96-3.17 (m, 4H), 4.01 (t, ³*J*_{H}-_{H} = 9.0 Hz, 4H), 4.28 (t, ³*J*_{H}-_{H}=6.0 Hz, 2H), 7.08-7.11 (d, 4 H), 7.20-7.35 (m, 10H), 7.45-7.48 (d, 4H), 8.37 (s, 6H) ppm).

### Example 9

### Synthesis of Di-p-toluenesulfonic Acid Salts of Bis(_{L}-phenylalanine)-Octane 1,8-Diester Monomer. (1-PHE-8).

Synthesis of di-*p*-toluene sulfonic acid of bis(L-phenylalanine)-octane 1,8-diester (1-PHE-8) was carried out using the method described in Example 7, above with 1,8 octanediol as the diol (157 g, 75% yield). The compound produced was characterized by ¹H NMR ((300 MHz, 303 K, DMSO-*d₆*): *δ* =1.14 (s, 8H) 1.41 (m, 4H), 2.27 (s, 6H), 2.50 (m, DMSO), 2.96-3.17 (m, 4H), 4.02 (t, ³*J*_{H}-_{H} 6.0 Hz,, 4H), 4.28 (t, ³*J*_{H}-_{H}=6.0 Hz, 2H) 7.08-7.11 (d, 4H) 7.20-7.35 (m, 10H) 7.48-7.49 (d, 4H) 8.36 (s, 6H) ppm).

### Example 10

### Synthesis of Di-p-toluenesulfonic Acid Salt of Bis-L-phenylalanine-octane-1,8-diester (1-PHE-8)

1,8-octanediol (10.00 g, 0.068 mol), *L*-phenylalanine (25.79 g, 0.156 mol), *p-*toluenesulfonic acid monohydrate (31.07 g, 0.163 mol) and toluene (200 mL) were mixed in a 500 mL round-bottom flask equipped with Dean-Stark trap and a magnetic stir bar. The system was heated to reflux for 20 h. After the reaction mixture was cooled to ambient temperature, the product was filtered and washed with diethyl ether. The solid product was dissolved in 3 L of hot water and decolored using activated carbon black (2.00 g) for 2-3 min. After hot filtration and cooling to room temperature, a white solid product was obtained by vacuum filtration. The product was then recrystallized with water for three times to yield 45.9 g (yield 86%) white powders as product. The compound produced was characterized by ¹H NMR ((500 MHz, DMSO-*d₆*): 1.08-1.22 (m, 8H), 1.37-1.49 (m, 4H), 2.29 (s, 6H), 3.02 (dd, *J* = 14.06, 7.95 Hz, 2H), 3.14 (dd, *J* = 13.94, 5.87 Hz, 2H), 3.98-4.08 (m, 4H), 4.28 (dd, *J* = 7.83, 6.11 Hz, 2H), 7.11 (dd, *J* = 8.44, 0.61 Hz, 4H), 7.20-7.36 (m, 10H), 7.48 (d, *J* = 7.83 Hz, 4H), 8.36 (br. s., 6H)) and ¹³C NMR ((125 MHz, DMSO-*d₆*): 21.25, 25.49, 28.20, 28.86, 36.65, 53.83, 65.96, 125.99, 127.65, 128.65, 128.97, 129.76, 135.18, 138.56, 145.46, 169.47).

### Example 11

### Synthesis of Di-p-toluenesulfonic Acid Salt of Bis-L-leucine-octane-1,8-diester (1-LEU-8)

1,8-octanediol (10.00 g, 0.068 mol), *L*-leucine (20.46 g, 0.156 mol), *p-*toluenesulfonic acid monohydrate (31.07 g, 0.163 mol) and toluene (200 mL) were mixed in a 500 mL round-bottom flask equipped with Dean-Stark trap and a magnetic stir bar. The system was heated to reflux for 20 h. After the reaction mixture was cooled to ambient temperature, the product was filtered and washed with diethyl ether. The solid product was recrystallized with water for three times to yield 42.9 g (yield 88%) white powder as the product. The compound produced was characterized by ¹H NMR ((300 MHz, DMSO-*d₆*): 0.89 (d, *J* = 5.86 Hz, 12H), 1.28 (br. s., 8H), 1.51-1.65 (m, 8H), 1.66-1.78 (m, 2H), 2.29 (s, 6H), 3.98 (t,*J* = 7.03 Hz, 2H), 4.07-4.23 (m, 4H), 7.12 (dd,*J* = 8.49, 0.59 Hz, 4H), 7.42-7.54 (m, 4H), 8.30 (br. s., 6H)) and by ¹³C NMR ((125 MHz, DMSO-*d₆*): 21.22, 22.37, 22.58, 24.26, 25.58, 28.32, 28.89, 51.09, 66.06, 125.93, 128.51, 138.18, 145.94, 170.38).

### Example 12

### General Procedure for Synthesis of Di-p-toluenesulfonic Acid Salts of Bis-L-alanine-diester monomers

Either 1,6-hexanediol, 1,8-octanediol, 1,10-decanediol, or 1,12-dodecandiol (1.0 mol equiv), *L*-alanine (2.3 mol equiv), p-toluenesulfonic acid monohydrate (TsOH) (2.4 mol equiv), and toluene (1 mL per gram of TsOH) were added to round-bottom flask equipped with Dean- Stark trap and condenser. The solution was heated to reflux (ca. 110 °C) while stirring with a magnetic stir bar. After ca. 20 h, the reaction mixture was cooled to ambient temperature. The resulting precipitate was collected by vacuum filtration. The solid product was dissolved in minimal hot water and decolored using a small amount of activated carbon black for 2-3 min. This solution was filtered to remove the carbon black and was left to cool to room temperature. The precipitate was then recrystallized three times using hot water to give the purified monomer.

### Example 13

### Synthesis of Di-p-toluenesulfonic Acid Salt of Bis-L-alanine-hexane-1,6-diester. (1-ALA-6)

The di-*p*-toluenesulfonic acid salt of bis-*L*-alanine-hexane-1,6-diester monomer (1-ALA-6) was prepared by following the general procedure described in Example 12, above, with the exception of the recrystallization procedure. The monomer was recrystallized four times from a 1:1 mixture (by volume) of ethanol and isopropanol. The monomer was prepared on a 145 mmol scale (based on the diol) and obtained with a 79% yield. The compound produced was characterized by ¹H NMR ((500 MHz, DMSO-d6, δ): 8.27 (s, 6H; NH₃), 7.49 (d, J = 8.0 Hz, 4H; Ar-H), 7.12 (d, J = 7.8 Hz, 4H; Ar-H), 4.16 (m, 4H; CH₂), 4.10 (q, J = 7.2 Hz, 2H; CH), 2.29 (s, 6H; CH₃), 1.61 (m, 4H; CH₂), 1.39 (d, J = 7.2 Hz, 6H; CH₃), 1.35 (m, 4H; CH₂)) and by ¹³C NMR ((126 MHz, DMSO-d6, δ): 169.92, 145.21, 137.89, 128.10, 125.46, 65.49, 47.93, 27.76, 24.71, 20.75, 15.70. IR (cm⁻¹): 1743 (-C-(CO)-O-)).

### Example 14

### Synthesis of Di-p-toluenesulfonic Acid Salt of Bis-L-alanine-octane-1,8-diester. (1-ALA-8)

The di-*p*-toluenesulfonic acid salt of bis-*L*-alanine-octane-1,8-diester monomer (1-ALA-8) was prepared by following the general procedure described in Example 12, above, with the exception of the recrystallization procedure. The monomer was recrystallized four times from a 1:1 mixture (by volume) of ethanol and isopropanol. The monomer was prepared on a 147 mmol scale (based on the diol) and obtained with a 79% yield. The compound produced was characterized by ¹H NMR ((500 MHz, DMSO-d6, δ): 8.27 (s, 6H; NH₃), 7.49 (d, J = 8.0 Hz, 4H; Ar-H), 7.12 (d, J = 7.8 Hz, 4H; Ar-H), 4.13 (m, 6H; CH₂ and CH), 2.29 (s, 6H; CH₃), 1.59 (m, 4H; CH₂), 1.39 (d, J = 7.2 Hz, 6H; CH₃), 1.32 (m, 8H; CH₂)0 and by ¹³C NMR ((126 MHz, DMSO-d6, δ): 169.92, 145.27, 137.83, 128.10, 125.45, 65.56, 47.92, 28.43, 27.87, 25.05, 20.71, 15.69. IR (cm⁻¹): 1749 (-C-(CO)-O-)).

### Example 15

### Synthesis of Di-p-toluenesulfonic Acid Salt of Bis-L-alanine-decane-1,10-diester. (1-ALA-10).

The di-*p*-toluenesulfonic acid salt of bis-*L*-alanine-decane-1,10-diester monomer (1-ALA-10) was prepared by following the general procedure described in Example 12, above, with the exception of the recrystallization procedure. The monomer was recrystallized four times from a 1:1 mixture (by volume) of ethanol and isopropanol. The monomer was prepared on a 132 mmol scale (based on the diol) and obtained with an 80% yield. The compound produced was characterized by ¹H NMR ((500 MHz, DMSO-d6, δ): 8.26 (s, 6H; NH₃), 7.49 (d, J = 8.0 Hz, 4H; Ar-H), 7.12 (d, J = 8.0 Hz, 4H; Ar-H), 4.14 (m, 6H; CH₂ and CH), 2.29 (s, 6H; CH₃), 1.60 (m, 4H; CH₂), 1.39 (d, J = 7.2 Hz, 6H; CH₃), 1.29 (m, 12H; CH2)) and by ¹³C NMR ((126 MHz, DMSO-d6, δ): 169.92, 145.32, 137.78, 128.05, 125.45, 65.58, 47.90, 28.81, 28.55, 27.89, 25.12, 20.73, 15.68. IR (cm⁻¹): 1736 (-C-(CO)-O-)).

### Example 16

### Synthesis of Di-p-toluenesulfonic Acid Salt of Bis-L-alanine-dodecane-1,12-diester. (1-ALA-12).

The di-*p*-toluenesulfonic acid salt of bis-*L*-alanine-dodecane-1,12-diester monomer (1-ALA-12) was prepared by following the general procedure described in Example 12, above, with the exception of the recrystallization procedure. The monomer was recrystallized four times from a 1:1 mixture (by volume) of ethanol and isopropanol. The monomer was prepared on a 145 mmol scale (based on the diol) and obtained with an 80% yield. The compound produced was characterized by ¹H NMR ((500 MHz, DMSO-d6, δ): 8.27 (s, 6H; NH₃), 7.49 (d, J = 7.5 Hz, 4H; Ar-H), 7.12 (d, J = 7.5 Hz, 4H; Ar-H), 4.13 (m, 6H; CH₂ and CH), 2.29 (s, 6H; CH₃), 1.59 (m, 4H; CH₂), 1.39 (d, J = 7.0 Hz, 6H; CH₃), 1.27 (m, 16H; CH₂)) and by ¹³C NMR (126 MHz, DMSO-d6, δ): 169.90, 145.21, 137.85, 128.07, 125.45, 65.57, 47.92, 28.93, 28.89, 28.58, 27.90, 25.13, 20.74, 15.67. IR (cm⁻¹): 1736 (-C-(CO)-O-)).

### Example 17

### Synthesis of bis(L-valine)-octane 1,8-diester based poly(ester urea) p(1-VAL-8).

The synthesis of 1-VAL-8 poly(ester urea) (PEU) was based on previously published procedures (See Schemes 1 and 2, above). In short, interfacial polymerization of 1-VAL-8 monomer was performed by dissolving the monomer (103.2 g, 0.15 mol, 1 equiv) and sodium carbonate (33.4 g, 0.31 mol, 2.1 equiv) in distilled water (1000 ml) in a 5 L 3-neck round-bottom flask. The solution was placed in a 40 °C water bath with overhead mechanical stirring until clear. The mixture was then cooled to 0 °C. In a separate container, additional sodium carbonate (16.7 g, 0.16 mol, 1.05 equiv) was dissolved in distilled water and added to the reaction flask, and the solution was allowed to stir until clear. Separately, triphosgene (15.8 g, 0.05 mol, 0.35 equiv) was dissolved in distilled chloroform (360 ml) and subsequently added to the reaction flask using an addition funnel. The solution turned white immediately and was allowed to stir for 30 min. An additional aliquot of triphosgene (3.6 g, 0.01 mol, 0.08 equiv) dissolved in distilled chloroform (0.15 M) was added to solution dropwise (~1 drop/second) using the addition funnel. The reaction was stirred for 24 h and then transferred to a separatory funnel and washed with water (3×). The organic phase was collected and precipitated in hot water to remove chloroform and residual starting material. The product was and dried under vacuum to obtain a white polymer (88% yield). (*M*_{w} = 28.4 kDa, *M*ₙ = 52.9 kDa, *Ð*_{M} = 1.86, Tg = 52.4 °C, *T*_{d} = 297 °C).

**p(1-VAL-8)** ¹H-NMR (300 MHz, DMSO-*d*₆, 30 °C) δ ppm 0.83 (m, 12H) 1.25 (s, 12H) 1.53 (s, 4H) 1.98 (m, 2H) 4.03 (m, 2H) 4.10 (m, 4H) 6.37 (d, 2H).

### Example 18

### Fabrication of PEU-Antibiotic Film.

Antibiotic (Cefazolin sodium) was first dissolved in methanol. P(1-VAL-8) was subsequently added and solutions were stirred at room temperature until PEU completely dissolved. Accordingly, PEU-Antibiotic (PEU-A) solutions containing 2 wt%, 5 wt % and 10 wt% of Cefazolin by weight with respect to p(1-VAL-8) were prepared. Film fabrication was done using blade assisted solution casting (FIGS. 1A-F). The PEU-A solution was poured into the well of a casting blade box. As the casting blade moves, the solution flows through the gap between the casting blade and PET substrate film. The substrate film gets coated with PEU-A solution which forms a uniform film. The thickness of the film was altered by changing the gap height or casting speed. PEU films did not adhere strongly to the substrate film and could be readily peeled off. The films were air-dried for 24 h and then vacuum-dried for another 48 h for complete solvent removal. Table 1 enumerates sample groups used in the study.

### Example 19

### Sink conditions for characterizing In vitro release.

Samples from each group were incubated in 3 mL PBS (pH 7.4) in glass vials at 37 °C under mild agitation of 70 rpm using a benchtop shaker. At each timepoint, 50 µL aliquots were withdrawn from the elution medium for determining antibiotic release. The amount of released antibiotic was then determined using an HPLC instrument. After collecting aliquots, the samples were moved to new vials and incubated under similar conditions.

### Example 20

### Content Uniformity testing.

Content uniformity testing was done to determine antibiotic distribution across the entire film and, to determine actual antibiotic content. For this, disks (diameter = 6mm) were punched out from a different section of each film, weighed and dissolved in 5ml of methanol. This was further diluted 10-fold with additional methanol. This diluted solution was used to determine cefazolin content in each sample using the HPLC method described below.

### Example 21

### Chromatographic conditions for analysis of samples.

Samples were chromatographed isocratically at 25°C, with a mobile phase consisting of acetonitrile (HPLC grade) and monobasic sodium phosphate monohydrate buffer (2.759 g in 1 L of purified water, pH adjusted to 2.5 ± 0.1 with phosphoric acid) at a ratio of 17:83 (v/v). The mobile phase was degassed prior to operation with a flow rate of 1.0 mL per minute, an injection volume of 20 µL and effluent was monitored at 270 nm.

The chromatography was performed on an Agilent 1290 Infinity II system equipped with a 1290 quaternary pump, 1290 vial sampler, and a 1290 Diode array detector. The peak areas were integrated automatically using the Empower software program and amounts were calculated from calibration curves. The chromatographic separation was carried out under isocratic reverse phase conditions on an Agilent^{™} InfinityLab Poroshell 120 EC-C18 (75 x 4.6 mm, 4 µm particle size) column and an InfinityLab Poroshell 120 EC-C18 (5 x 4.6 mm, 4 µm particle size) was used as guard column.

### Example 22

### Characterizing Antimicrobial susceptibility.

To assess the antimicrobial activity of antibiotic released from PEU-A films diffusion assays and a broth incubation assay were performed using *S. aureus* (ATCC 25923). Samples for bacterial studies were prepared by punching out 6 mm diameter circular disks. The samples were EtO (ethylene oxide) sterilized and kept under vacuum for 72 h to remove any residual EtO.

### Example 23

### Disk-diffusion assay.

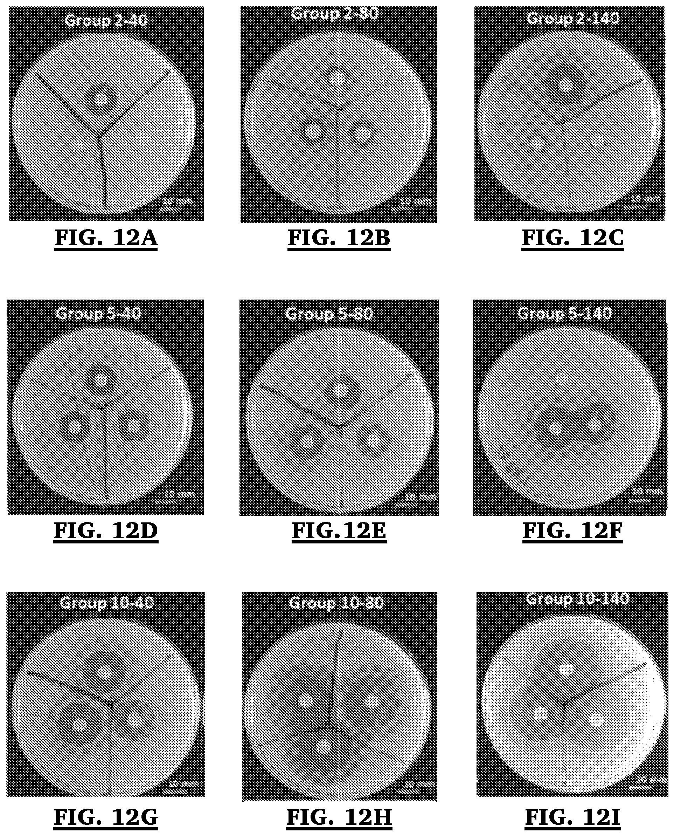

The procedure was adapted from Clinical and Laboratory Standards Institute (CLSI) standards and previously published literature. For preparing inoculum, a single colony of the S. *aureus* was grown overnight in Luria-Bertani broth at 37°C under constant shaking of 250 rpm. The overnight culture was diluted with broth to obtain an OD = 0.09. Antimicrobial activity was evaluated by measuring the diameter of the inhibition zone around the PEU-A disks. For disk-diffusion testing, 90-mm-diameter Petri dishes containing MH agar at a depth of 4-5 mm were used. Bacterial culture was inoculated on the agar surface using a sterile cotton swab. After streaking a confluent lawn, PEU-A disks were placed onto the inoculated agar. The Petri dishes were incubated at 37°C for 24 h. After 24 h, the diameters of inhibition zone (mm) were measured in four perpendicular directions by digital calipers. FIGS. 12A-I are images showing the results of the disc-diffusion assays for all 9 groups of PEU-A films. Triplicates of each group were used and the mean zone size was determined.

### Example 24

### Well-diffusion assay.

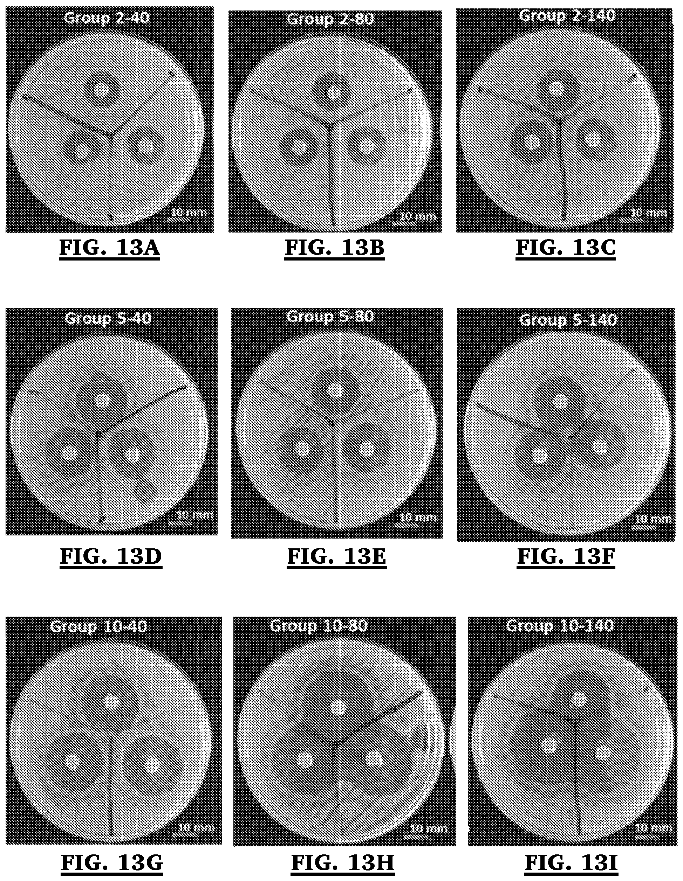

The method was adapted from previously published literature. Agar plates were prepared similarly to the disk-diffusion method. After inoculation of bacterial culture, a sterile 6 mm biopsy punch was used to aseptically create a well in the agar. Then, PEU-A disks were aseptically placed in the wells using sterile tweezers. Also, 80µL of MH broth was added to the well. After 24 h of incubation under similar conditions as above, diameters of the inhibition zone (mm) were measured in four perpendicular directions. FIGS. 13A-I are images showing the results of the well-diffusion assays for all 9 groups of PEU-A films. Each test was performed on all groups in triplicate and the mean zone size was determined.

### Example 25

### Broth incubation assay.

For preparing inoculum, a single colony of the S. *aureus* was grown overnight in MH broth at 37°C under constant shaking of 250 rpm. The culture was diluted with LB broth to obtain an inoculum with a CFU/ml of 1.5 X 10³. In a 96-well microtiter plate, the sample-wells were filled with 40 µL of this inoculum, 160 µL of LB broth and PEU-A disks from each group were placed in them. Positive-control well contained 200 µL of broth and PEU-A disks in them. Negative Control-1 wells contained 160 µL of LB broth and 40 µL of inoculum. Negative Control-2 wells contained 160 µL of LB broth, 40 µL of inoculum, and blank PEU disks without any antibiotic. The plate was incubated in at 37 °C (5% CO2) for 24 h with constant agitation at 80 rpm. After 24 h of incubation, the disks were removed, and the CFU in each well was enumerated via 10-fold series dilution and plating 100 µL onto LB agar, incubating overnight and then plate counting CFU. The mean CFU for each group was calculated by accounting for dilutions.

### Example 26

### ¹H NMR characterization

The chemical structure of the p(1-VAL-8) PEU polymer of Example 17 above was characterized using ¹H-NMR spectroscopy and FTIR. The ¹H-NMR was performed on a Varian Mercury 300 MHz Spectrometer, in deuterated dimethyl sulfoxide (DMSO-*d*₆) at 25 °C. The chemical shifts, δ (ppm), were referenced to residual solvent resonance frequencies (¹H-NMR DMSO-*d*₆ 2.50 ppm). (See, FIG. 3)

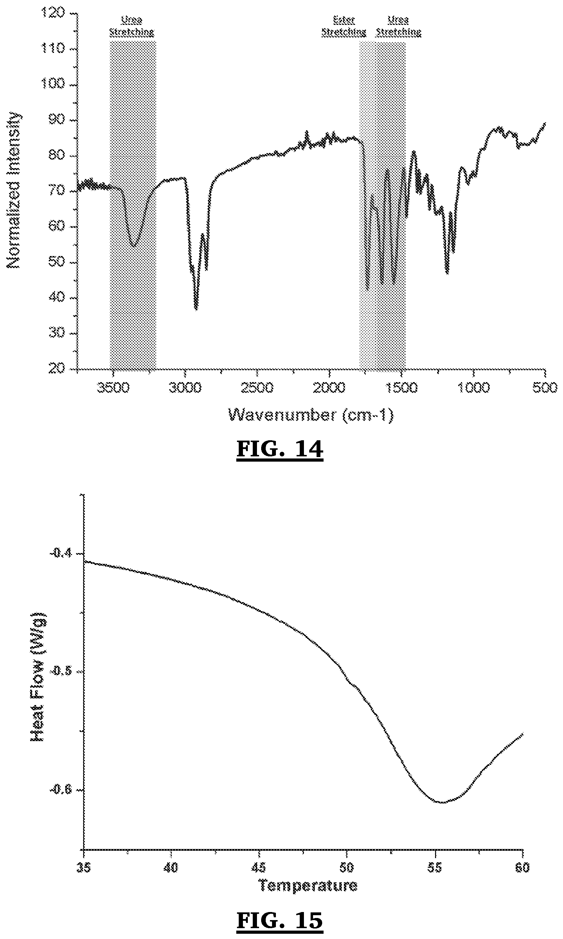

In the ATR-FTIR spectrum in FIG. 14, the main peaks are labeled and the values of those and others are listed below. The broad peak that appears at 3350-3500 cm⁻¹ was the N-H (urea) stretching peak. The peak at 1735 cm⁻¹ is characteristic of the C=O (ester) bond stretching and confirms the presence of an ester bond. The peak at 1650-1690 cm⁻¹ is characteristic of the C=O (urea) bond stretching from the urea and confirms the presence of the urea bond. The peak at 1000-1300 cm⁻¹ is characteristic of the C-O (ester) bond and the C-H (alkyl) bending. (See, FIG. 14) All these characteristic signals confirm that we had successfully synthesized the p(1-VAL-8) PEU.

### Example 27

### Thermal characterization

The degradation temperature (T_{d}) of the p(1-VAL-8) PEU polymer of Example 17 was obtained by TGA across a temperature range of 0°C to 800°C at a scanning rate of 20°C/min under nitrogen. The glass transition temperature (T_{g}) was determined using differential scanning calorimeter (DSC) by temperature sweep from -10 °C to 120 °C at a scanning rate of 10°C/min. The DSC used was a TA Q200, while TGA was done on TA Q50.

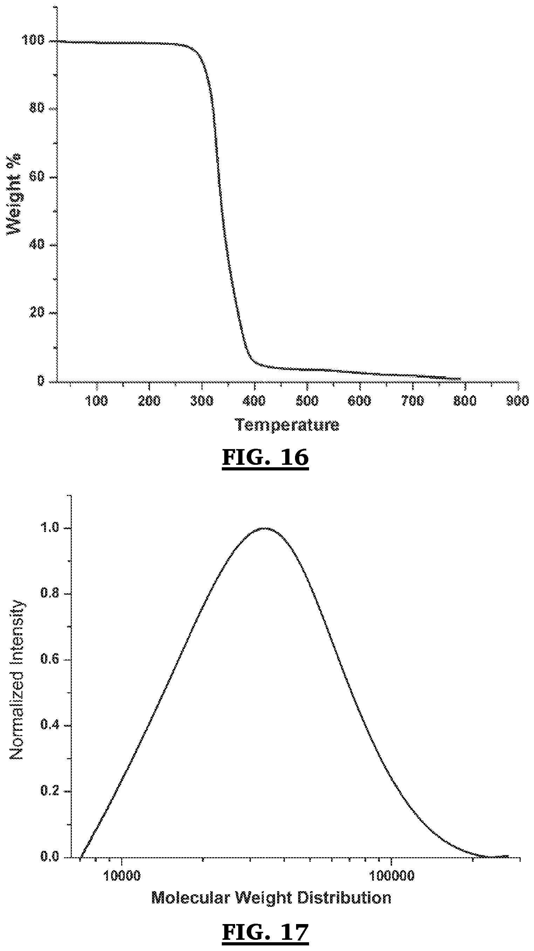

The data obtained from the DSC curve (FIG. 15) was used to calculate the glass transition temperature (*T*_{g}) of the polymer. The *T*_{g} calculated using the curve was 52.4 °C. The TGA curve (See, FIG. 16) was used to calculate the degradation temperature (*T*_{d}) of the polymer. The *T*_{d} calculated using the curve above was 297 °C. The molecular mass values of the p(1-VAL-8) PEU polymer were calculated from the SEC chromatogram (FIG. 17) using the EcoSEC software (*M*_{w} = 52,900 g/mol, *M*ₙ = 28,400 g/mol and *Ð*ₘ = 1.86). The *M*_{w} has a wide range because step-growth polymerization was used for synthesizing this polymer. The *Ð*ₘ for these types of reactions is expected to be from 1.8 to 2.2.

In light of the foregoing, it should be appreciated that the present invention significantly advances the art by providing a drug-loaded degradable amino acid based poly(ester urea) film that is structurally and functionally improved in a number of ways. While particular embodiments of the invention have been disclosed in detail herein, it should be appreciated that the invention is not limited thereto or thereby. The scope of the invention shall be defined by the claims that follow.

## Claims

1. A drug loaded degradable amino acid based poly(ester urea) film for drug delivery comprising:
an amino acid based poly(ester urea) polymer comprising residues of two or more amino acid based diester monomer segments, wherein said two or more amino acid based diester monomer segments comprise the residues of two amino acids selected from the group consisting of alanine (ala - A ); arginine (arg - R); asparagine (asn - N); aspartic acid (asp - D); cysteine (cys - C); glutamine (gln - Q); glutamic acid (glu - E); glycine (gly - G); histidine (his - H); isoleucine (ile - I); leucine (leu - L); lysine (lys - K); methionine (met - M); phenylalanine (phe - F); serine (ser - S); threonine (thr - T); tryptophan (trp - W); tyrosine (tyr - Y); valine (val - V) and combinations thereof separated by from 1 to 20 carbon atoms and are joined by urea linkages to form said an amino acid based poly(ester urea) polymer; and
a drug,
wherein said drug comprises from 1 weight percent to 50 weight percent of the drug loaded degradable amino acid based poly(ester urea) film;
wherein said drug loaded degradable amino acid based poly(ester urea) film has a thickness of from 1µm to 1000 µm, preferably from 10 µm to 500 µm, and more preferably from 10 µm to 150 µm.

2. The drug loaded degradable amino acid based poly(ester urea) film of claim 1 wherein said two amino acids are both L-valine.

3. The drug loaded degradable amino acid based poly(ester urea) film of claim 1 wherein the degradable amino acid based poly(ester urea) polymer has the formula: 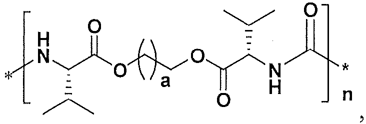 or 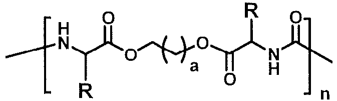 where R is -CH₃, -(CH₂)₃NHC(NH₂)C=NH, -CH₂CONH₂,-CH₂COOH, -CH₂SH, - (CH₂)₂COOH, -(CH₂)₂CONH₂, -NH₂, -CH₂C=CH-N=CH-NH, -CH(CH₃)CH₂CH₃, - CH₂CH(CH₃)₂, -(CH₂)₄NH₂, -(CH₂)₂SCH₃, -CH₂Ph, -CH₂OH, -CH(OH)CH₃, -CH₂-C=CH-NH-Ph, -CH₂-Ph-OH,-CH(CH₃)₂, or -CH₂C₆H₄OCH₂C₆H₅; a is an integer from 1 to 19 and n is an integer from 20 to 300.

4. The drug loaded degradable amino acid based poly(ester urea) film of claim 1 wherein said drug is selected from the group consisting of antibiotics, cancer drugs, antipsychotics, antidepressants, sleep aids, tranquillizers, anti-Parkinson's drugs, mood stabilizers, pain killers, anti-inflammatories, anti-microbials, and combinations thereof or an antibiotic selected from the group consisting of lipopeptides, fluoroquinolone, lipoglycopeptides, cephalosporins, penicillins, monobactams, carbapenems, macrolide antibiotics, lincosamides, streptogramins, aminoglycoside antibiotics, quinolone antibiotics, sulfonamides, tetracycline antibiotics, chloraphenicol, metronidazole, tinidazole, nitrofurantoin, glycopeptides, oxazolidinones, rifamycins, polypeptides, tuberactinomycins, and combinations or pharmaceutically acceptable salts thereof.

5. The drug loaded degradable amino acid based poly(ester urea) film of claim 1 wherein said drug is a tetracycline antibiotic, rifamycin antibiotic, cefazolin sodium or a pharmaceutically acceptable salt thereof.

6. The drug loaded degradable amino acid based poly(ester urea) film of claim 1 comprising from 1 weight percent to 10 weight percent, preferably from 2 weight percent to 10 weight percent of said drug and more preferably from 1 weight percent to 5 weight percent of said drug.

7. The drug loaded degradable amino acid based poly(ester urea) film of claim 1 wherein the drug is uniformly distributed throughout said degradable amino acid based poly(ester urea) polymer.

8. An implantable medical device comprising the drug loaded degradable amino acid based poly(ester urea) film of any of claims 1-7.

9. The implantable medical device of claim 8 wherein said implantable medical device comprises a cardiac implantable electronic device (CIED).

10. A method of making the drug loaded degradable amino acid based poly(ester urea) film for drug delivery of claim 1 comprising:
A) preparing a suitable substrate;
B) preparing a degradable amino acid based poly(ester urea) polymer comprising residues of two or more amino acid based diester monomer segments, wherein said two or more amino acid based diester monomer segments comprise the residues of two amino acids selected from the group consisting of alanine (ala - A); arginine (arg - R); asparagine (asn - N); aspartic acid (asp - D); cysteine (cys - C); glutamine (gln - Q); glutamic acid (glu - E); glycine (gly - G); histidine (his - H); isoleucine (ile - I); leucine (leu - L); lysine (lys - K); methionine (met - M); phenylalanine (phe - F); serine (ser - S); threonine (thr - T); tryptophan (trp - W); tyrosine (tyr - Y); valine (val - V) and combinations thereof separated by from 1 to 20 carbon atoms and are joined by urea linkages to form said an amino acid based poly(ester urea) polymer;
C) selecting a drug to be delivered from the group consisting of antibiotics, cancer drugs, antipsychotics, antidepressants, sleep aids, tranquillizers, anti-Parkinson's drugs, mood stabilizers, pain killers, anti-inflammatories, anti-microbials, and combinations thereof and dissolving it in a co-solvent for the drug and the degradable amino acid based poly(ester urea) polymer.
D) dissolving the degradable amino acid based poly(ester urea) polymer in the drug solution; and
E) forming the amino acid based poly(ester urea) polymer and drug solution of step (D) into a film on the substrate, wherein said drug comprises from 1 weight percent to 50 weight percent of the drug loaded degradable amino acid based poly(ester urea) film and wherein said film has a thickness of from 1µm to 1000 µm, preferably from 10 µm to 500 µm, and more preferably from 10 µm to 150 µm.

11. The method of claim 10 wherein the substrate comprises an implantable medical device.

12. The method of claim 10 wherein said two amino acids are both L-valine or the drug to be delivered (step C) is a tetracycline antibiotic, a rifamycin antibiotic, cefazolin sodium or a pharmaceutically acceptable salt thereof.

13. The method of claim 10 wherein said drug to be delivered (step C) is selected from the group consisting of antibiotics, cancer drugs, antipsychotics, antidepressants, sleep aids, tranquillizers, anti-Parkinson's drugs, mood stabilizers, pain killers, anti-inflammatories, anti-microbials, and combinations thereof or the drug to be delivered (step C) is an antibiotic selected from the group consisting of lipopeptides, fluoroquinolone, lipoglycopeptides, cephalosporins, penicillins, monobactams, carbapenems, macrolide antibiotics, lincosamides, streptogramins, aminoglycoside antibiotics, quinolone antibiotics, sulfonamides, tetracycline antibiotics, chloraphenicol, metronidazole, tinidazole, nitrofurantoin, glycopeptides, oxazolidinones, rifamycins, polypeptides, tuberactinomycins, and combinations and pharmaceutically acceptable salts thereof.

14. The method of claim 10 wherein the ratio of the degradable amino acid based poly(ester urea) polymer to the drug in the solution of step (D) is from 100:1 to 1:1, preferably from 10:1 to 1:1; and more preferably from 5:1 to 1:1 by weight or the step of forming a film (Step E) is performed by blade coating, solvent coating, lamination, spray coating, extruding, injection molding, or a combination thereof.

15. The drug loaded degradable amino acid based poly(ester urea) film of claim 1 wherein said film is formed into an envelope, pouch, or pocket, that is sized to receive an implantable medical device.

## Patentansprüche

1. Wirkstoffbeladener abbaubarer auf Aminosäuren basierender Poly(esterharnstoff)-Film für eine Wirkstoffverabreichung umfassend:
ein auf Aminosäure basierendes Poly(esterharnstoff)-Polymer, umfassend Reste von zwei oder mehr auf Aminosäure basierenden Diestermonomersegmenten, wobei die zwei oder mehr auf Aminosäure basierenden Diestermonomersegmente die Reste von zwei Aminosäuren umfassen, die aus der Gruppe ausgewählt sind, bestehend aus Alanin (ala - A); Arginin (arg - R); Asparagin (asn - N); Asparaginsäure (asp - D); Cystein (cys - C); Glutamin (gln - Q); Glutaminsäure (glu - E); Glycin (gly - G); Histidin (his - H); Isoleucin (ile - I); Leucin (leu - L); Lysin (lys - K); Methionin (met - M); Phenylalanin (phe - F); Serin (ser - S); Threonin (thr - T); Tryptophan (trp - W); Tyrosin (tyr - Y); Valin (val - V) und Kombinationen davon, getrennt durch 1 bis 20 Kohlenstoffatome, und durch Harnstoffbindungen verbunden sind, um das aminosäurebasierte Poly(esterharnstoff)-Polymer auszubilden; und
einen Wirkstoff,
wobei der Wirkstoff zu 1 Gewichtsprozent bis 50 Gewichtsprozent den wirkstoffbeladenen abbaubaren auf Aminosäure basierenden Poly(esterharnstoff)-Film umfasst;
wobei der wirkstoffbeladene abbaubare auf Aminosäure basierende Poly(esterharnstoff)-Film eine Dicke von 1 µm bis 1000 µm, vorzugsweise von 10 µm bis 500 µm und stärker bevorzugt von 10 µm bis 150 µm aufweist.

2. Wirkstoffbeladener abbaubarer auf Aminosäuren basierender Poly(esterharnstoff)-Film nach Anspruch 1, wobei die zwei Aminosäuren beide L-Valin sind.

3. Wirkstoffbeladener abbaubarer auf Aminosäure basierender Poly(esterharnstoff)-Film nach Anspruch 1, wobei das abbaubare auf Aminosäure basierende Poly(esterharnstoff)-Polymer die Formel aufweist: 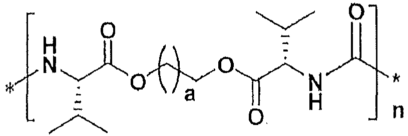 oder 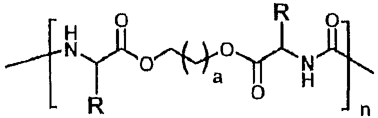 wobei R -CH₃, -(CH₂)₃NHC(NH₂)C=NH, -CH₂CONH₂-CH₂COOH, -CH₂SH, -(CH₂)₂COOH, -(CH₂)₂CONH₂, -NH₂, -CH₂C=CH-N=CH-NH, -CH(CH₃)CH₂CH₃, -CH₂CH(CH₃)₂, -(CH₂)₄NH₂, - (CH₂)₂SCH₃, -CH₂Ph, -CH₂OH, -CH(OH)CH₃, -CH₂-C=CH-NH-Ph, -CH₂-Ph-OH, -CH(CH₃)₂ oder -CH₂C₆H₄OCH₂C₆H₅ ist; a eine ganze Zahl von 1 bis 19 ist und n eine ganze Zahl von 20 bis 300 ist.

4. Wirkstoffbeladener abbaubarer auf Aminosäuren basierender Poly(esterharnstoff)-Film nach Anspruch 1, wobei der Wirkstoff aus der Gruppe ausgewählt ist, bestehend aus Antibiotika, Krebswirkstoffen, Antipsychotika, Antidepressiva, Schlafmitteln, Beruhigungsmitteln, Anti-Parkinson-Wirkstoffen, Stimmungsstabilisatoren, Schmerzmitteln, Entzündungshemmern, antimikrobiellen Mitteln und Kombinationen davon, oder ein Antibiotikum ist, das aus der Gruppe ausgewählt ist, bestehend aus Lipopeptiden, Fluorchinolon, Lipoglykopeptiden, Cephalosporinen, Penicillinen, Monobactamen, Carbapenemen, Makrolid-Antibiotika, Lincosamiden, Streptograminen, Aminoglykosid-Antibiotika, Chinolon-Antibiotika, Sulfonamiden, Tetracyclin-Antibiotika, Chloramphenicol, Metronidazol, Tinidazol, Nitrofurantoin, Glykopeptiden, Oxazolidinonen, Rifamycinen, Polypeptiden, Tuberactinomycinen und Kombinationen oder pharmazeutisch geeigneten Salzen davon.

5. Wirkstoffbeladener abbaubarer auf Aminosäuren basierender Poly(esterharnstoff)-Film nach Anspruch 1, wobei der Wirkstoff ein Tetracyclin-Antibiotikum, Rifamycin-Antibiotikum, Cefazolin-Natrium oder ein pharmazeutisch geeignetes Salz davon ist.

6. Wirkstoffbeladener abbaubarer auf Aminosäuren basierender Poly(esterharnstoff)-Film nach Anspruch 1, umfassend zu 1 Gewichtsprozent bis 10 Gewichtsprozent, vorzugsweise zu 2 Gewichtsprozent bis 10 Gewichtsprozent den Wirkstoff und stärker bevorzugt zu 1 Gewichtsprozent bis 5 Gewichtsprozent den Wirkstoff.

7. Wirkstoffbeladener abbaubarer auf Aminosäuren basierender Poly(esterharnstoff)-Film nach Anspruch 1, wobei der Wirkstoff gleichmäßig in dem abbaubaren auf Aminosäure basierenden Poly(esterharnstoff)-Polymer verteilt ist.

8. Implantierbare medizinische Vorrichtung, umfassend den wirkstoffbeladenen abbaubaren auf Aminosäure basierenden Poly(esterharnstoff)-Film nach einem der Ansprüche 1 bis 7.

9. Implantierbare medizinische Vorrichtung nach Anspruch 8, wobei die implantierbare medizinische Vorrichtung eine kardiale implantierbare elektronische Vorrichtung (CIED) umfasst.

10. Verfahren zum Herstellen des wirkstoffbeladenen abbaubaren auf Aminosäure basierenden Poly(esterharnstoff)-Films für die Wirkstoffverabreichung nach Anspruch 1, umfassend:
A) Bereitstellen eines geeigneten Substrats;
B) Zubereiten eines abbaubaren auf Aminosäure basierenden Poly(esterharnstoff)-Polymers, das Reste von zwei oder mehr auf Aminosäure basierenden Diestermonomersegmenten umfasst, wobei die zwei oder mehr auf Aminosäure basierenden Diestermonomersegmente die Reste von zwei Aminosäuren umfassen, die aus der Gruppe ausgewählt sind, bestehend aus Alanin (ala - A); Arginin (arg - R); Asparagin (asn - N); Asparaginsäure (asp - D); Cystein (cys - C); Glutamin (gln - Q); Glutaminsäure (glu - E); Glycin (gly - G); Histidin (his - H); Isoleucin (ile - I); Leucin (leu - L); Lysin (lys - K); Methionin (met - M); Phenylalanin (phe - F); Serin (ser - S); Threonin (thr - T); Tryptophan (trp - W); Tyrosin (tyr - Y); Valin (val - V) und Kombinationen davon, getrennt durch 1 bis 20 Kohlenstoffatome, und durch Harnstoffbindungen verbunden sind, um das aminosäurebasierte Poly(esterharnstoff)-Polymer auszubilden;
C) Auswählen eines zu verabreichenden Wirkstoffs aus der Gruppe, bestehend aus Antibiotika, Krebswirkstoffen, Antipsychotika, Antidepressiva, Schlafmitteln, Beruhigungsmitteln, Anti-Parkinson-Wirkstoffen, Stimmungsstabilisatoren, Schmerzmitteln, Entzündungshemmern, antimikrobiellen Mitteln und Kombinationen davon und Auflösen davon in einem Co-Lösungsmittel für den Wirkstoff und das abbaubare auf Aminosäure basierende Poly(esterharnstoff)-Polymer.
D) Auflösen des abbaubaren auf Aminosäure basierenden Poly(esterharnstoff)-Polymers in der Wirkstofflösung; und
E) Ausbilden der Lösung aus auf Aminosäure basierendem Poly(esterharnstoff)-Polymer und Wirkstoff aus Schritt (D) zu einem Film auf dem Substrat, wobei der Wirkstoff zu 1 Gewichtsprozent bis 50 Gewichtsprozent den wirkstoffbeladenen abbaubaren auf Aminosäure basierenden Poly(esterharnstoff)-Film umfasst und wobei der Film eine Dicke von 1 µm bis 1000 µm, vorzugsweise von 10 µm bis 500 µm und stärker bevorzugt von 10 µm bis 150 µm aufweist.

11. Verfahren nach Anspruch 10, wobei das Substrat eine implantierbare medizinische Vorrichtung umfasst.

12. Verfahren nach Anspruch 10, wobei die zwei Aminosäuren beide L-Valin sind oder der zu verabreichende Wirkstoff (Schritt C) ein Tetracyclin-Antibiotikum, ein Rifamycin-Antibiotikum, Cefazolin-Natrium oder ein pharmazeutisch geeignetes Salz davon ist.

13. Verfahren nach Anspruch 10, wobei der zu verabreichende Wirkstoff (Schritt C) aus der Gruppe ausgewählt ist, bestehend aus Antibiotika, Krebswirkstoffen, Antipsychotika, Antidepressiva, Schlafmitteln, Beruhigungsmitteln, Anti-Parkinson-Wirkstoffen, Stimmungsstabilisatoren, Schmerzmitteln, Entzündungshemmern, Antimikrobiellen und Kombinationen davon, oder der zu verabreichende Wirkstoff (Schritt C) ein Antibiotikum ist, das aus der Gruppe ausgewählt ist, bestehend aus Lipopeptiden, Fluorchinolon, Lipoglykopeptiden, Cephalosporinen, Penicillinen, Monobactamen, Carbapenemen, Makrolid-Antibiotika, Lincosamiden, Streptograminen, Aminoglykosid-Antibiotika, Chinolon-Antibiotika, Sulfonamiden, Tetracyclin-Antibiotika, Chloramphenicol, Metronidazol, Tinidazol, Nitrofurantoin, Glykopeptiden, Oxazolidinonen, Rifamycinen, Polypeptiden, Tuberactinomycinen und Kombinationen und pharmazeutisch geeigneten Salzen davon.

14. Verfahren nach Anspruch 10, wobei das Verhältnis des abbaubaren auf Aminosäure basierenden Poly(esterharnstoff)-Polymers zu dem Wirkstoff in der Lösung von Schritt (D) von 100 : 1 bis 1 : 1, vorzugsweise von 10 : 1 bis 1 : 1; und stärker bevorzugt von 5 : 1 bis 1 : 1 nach Gewicht beträgt oder der Schritt des Ausbildens eines Films (Schritt E) durch Rakelbeschichtung, Lösungsmittelbeschichtung, Laminierung, Sprühbeschichtung, Extrudieren, Spritzgießen oder eine Kombination davon durchgeführt wird.

15. Wirkstoffbeladener abbaubarer auf Aminosäuren basierender Poly(esterharnstoff)-Film nach Anspruch 1, wobei der Film zu einer Hülle, einem Beutel oder einer Tasche geformt ist, die/der bemessen ist, um eine implantierbare medizinische Vorrichtung aufzunehmen.

## Revendications

1. Film dégradable de poly(ester-urée) à base d'acides aminés chargé de médicament pour délivrance de médicament comprenant :
un polymère poly(ester-urée) à base d'acides aminés comprenant des résidus de deux segments monomères diester à base d'acides aminés ou plus, dans lequel lesdits deux segments monomères diester à base d'acides aminés ou plus comprennent les résidus de deux acides aminés choisis dans le groupe constitué d'alanine (ala - A) ; arginine (arg - R) ; asparagine (asn - N) ; acide aspartique (asp - D) ; cystéine (cys - C) ; glutamine (gln - Q) ; acide glutamique (glu - E) ; glycine (gly - G) ; histidine (his - H) ; isoleucine (ile - I) ; leucine (leu - L) ; lysine (lys - K) ; méthionine (met - M) ; phénylalanine (phe - F) ; sérine (ser - S) ; thréonine (thr - T) ; tryptophane (trp - W) ; tyrosine (tyr - Y) ; valine (val - V) et des combinaisons de ceux-ci, séparés par 1 à 20 atomes de carbone et sont joints par des liaisons urée pour former ledit polymère poly(ester-urée) à base d'acides aminés ; et
un médicament,
dans lequel ledit médicament constitue de 1 pour cent en poids à 50 pour cent en poids du film dégradable de poly(ester-urée) à base d'acides aminés chargé de médicament ;
dans lequel ledit film dégradable de poly(ester-urée) à base d'acides aminés chargé de médicament a une épaisseur allant de 1 µm à 1000 µm, de préférence de 10 µm à 500 µm, et plus préférablement de 10 µm à 150 µm.

2. Film dégradable de poly(ester-urée) à base d'acides aminés chargé de médicament selon la revendication 1 dans lequel lesdits deux acides aminés sont l'un et l'autre L-valine.

3. Film dégradable de poly(ester-urée) à base d'acides aminés chargé de médicament selon la revendication 1 dans lequel le polymère poly(ester-urée) à base d'acides aminés dégradable a la formule : 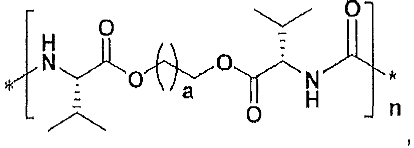 ou 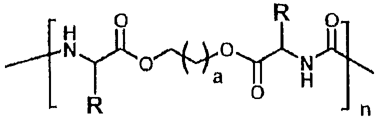 où R est -CH₃, -(CH₂)₃NHC(NH₂)C=NH, -CH₂CONH₂-CH₂COOH, -CH₂SH, - (CH₂)₂COOH, -(CH₂)₂CONH₂, -NH₂, -CH₂C=CH-N=CH-NH, -CH(CH₃)CH₂CH₃, - CH₂CH(CH₃)₂, -(CH₂)₄NH₂, -(CH₂)₂SCH₃, -CH₂Ph, -CH₂OH, -CH(OH)CH₃, -CH₂-C=CH- NH-Ph, -CH₂-Ph-OH,-CH(CH₃)₂ ou -CH₂C₆H₄OCH₂C₆H₅ ; a est un nombre entier allant de 1 à 19 et n est un nombre entier allant de 20 à 300.

4. Film dégradable de poly(ester-urée) à base d'acides aminés chargé de médicament selon la revendication 1 dans lequel ledit médicament est choisi dans le groupe constitué d'antibiotiques, médicaments anticancéreux, antipsychotiques, antidépresseurs, somnifères, tranquillisants, médicaments antiparkinsoniens, stabilisateurs de l'humeur, analgésiques, anti-inflammatoires, antimicrobiens, et combinaisons de ceux-ci ou un antibiotique choisi dans le groupe constitué de lipopeptides, fluoroquinolones, lipoglycopeptides, céphalosporines, pénicillines, monobactames, carbapénèmes, antibiotiques macrolides, lincosamides, streptogramines, antibiotiques aminoglycosides, antibiotiques quinolones, sulfamides, antibiotiques tétracyclines, chloramphénicol, métronidazole, tinidazole, nitrofurantoïne, glycopeptides, oxazolidinones, rifamycines, polypeptides, tuberactinomycines, et combinaisons ou sels pharmaceutiquement acceptables de ceux-ci.

5. Film dégradable de poly(ester-urée) à base d'acides aminés chargé de médicament selon la revendication 1 dans lequel ledit médicament est un antibiotique tétracycline, un antibiotique rifamycine, de la céfazoline sodique ou un sel pharmaceutiquement acceptable de ceux-ci.

6. Film dégradable de poly(ester-urée) à base d'acides aminés chargé de médicament selon la revendication 1 comprenant de 1 pour cent en poids à 10 pour cent en poids, de préférence de 2 pour cent en poids à 10 pour cent en poids dudit médicament et plus préférablement de 1 pour cent en poids à 5 pour cent en poids dudit médicament.

7. Film dégradable de poly(ester-urée) à base d'acides aminés chargé de médicament selon la revendication 1 dans lequel le médicament est uniformément réparti sur l'ensemble dudit polymère dégradable poly(ester-urée) à base d'acides aminés.

8. Dispositif médical implantable comprenant le film dégradable de poly(ester-urée) à base d'acides aminés chargé de médicament selon l'une quelconque des revendications 1 à 7.

9. Dispositif médical implantable selon la revendication 8 dans lequel ledit dispositif médical implantable comprend un dispositif électronique implantable cardiaque (CIED).

10. Procédé de fabrication du film dégradable de poly(ester-urée) à base d'acides aminés chargé de médicament pour délivrance de médicament selon la revendication 1 comprenant :
A) la préparation d'un substrat approprié ;
B) la préparation d'un polymère dégradable poly(ester-urée) à base d'acides aminés comprenant des résidus de deux segments monomères diester à base d'acides aminés ou plus, dans lequel lesdits deux segments monomères diester à base d'acides aminés ou plus comprennent les résidus de deux acides aminés choisis dans le groupe constitué d'alanine (ala - A) ; arginine (arg - R) ; asparagine (asn - N) ; acide aspartique (asp - D) ; cystéine (cys - C) ; glutamine (gln - Q) ; acide glutamique (glu - E) ; glycine (gly - G) ; histidine (his - H) ; isoleucine (ile - I) ; leucine (leu - L) ; lysine (lys - K) ; méthionine (met - M) ; phénylalanine (phe - F) ; sérine (ser - S) ; thréonine (thr - T) ; tryptophane (trp - W) ; tyrosine (tyr - Y) ; valine (val - V) et des combinaisons de ceux-ci, séparés par 1 à 20 atomes de carbone et sont joints par des liaisons urée pour former ledit polymère poly(ester-urée) à base d'acides aminés ;
C) la sélection d'un médicament à délivrer dans le groupe constitué d'antibiotiques, médicaments anticancéreux, antipsychotiques, antidépresseurs, somnifères, tranquillisants, médicaments antiparkinsoniens, stabilisateurs de l'humeur, analgésiques, anti-inflammatoires, antimicrobiens, et combinaisons de ceux-ci et sa dissolution dans un cosolvant pour le médicament et le polymère dégradable poly(ester-urée) à base d'acides aminés.
D) la dissolution du polymère dégradable poly(ester-urée) à base d'acides aminés dans la solution de médicament ; et
E) la formation du polymère poly(ester-urée) à base d'acides aminés et de la solution de médicament de l'étape (D) en un film sur le substrat, dans lequel ledit médicament comprend de 1 pour cent en poids à 50 pour cent en poids du film dégradable de poly(ester-urée) à base d'acides aminés chargé de médicament et dans lequel ledit film a une épaisseur allant de 1 µm à 1000 µm, de préférence de 10 µm à 500 µm, et plus préférablement de 10 µm à 150 µm.

11. Procédé selon la revendication 10 dans lequel le substrat comprend un dispositif médical implantable.

12. Procédé selon la revendication 10 dans lequel lesdits deux acides aminés sont l'un et l'autre L-valine ou le médicament à délivrer (étape C) est un antibiotique tétracycline, un antibiotique rifamycine, de la céfazoline sodique ou un sel pharmaceutiquement acceptable de ceux-ci.

13. Procédé selon la revendication 10 dans lequel ledit médicament à délivrer (étape C) est choisi dans le groupe constitué d'antibiotiques, médicaments anticancéreux, antipsychotiques, antidépresseurs, somnifères, tranquillisants, médicaments antiparkinsoniens, stabilisateurs de l'humeur, analgésiques, anti-inflammatoires, antimicrobiens, et combinaisons de ceux-ci ou le médicament à délivrer (étape C) est un antibiotique choisi dans le groupe constitué de lipopeptides, fluoroquinolones, lipoglycopeptides, céphalosporines, pénicillines, monobactames, carbapénèmes, antibiotiques macrolides, lincosamides, streptogramines, antibiotiques aminoglycosides, antibiotiques quinolones, sulfamides, antibiotiques tétracyclines, chloramphénicol, métronidazole, tinidazole, nitrofurantoïne, glycopeptides, oxazolidinones, rifamycines, polypeptides, tuberactinomycines, et combinaisons et sels pharmaceutiquement acceptables de ceux-ci.

14. Procédé selon la revendication 10 dans lequel le rapport du polymère dégradable poly(ester-urée) à base d'acides aminés au médicament dans la solution de l'étape (D) va de 100:1 à 1:1, de préférence de 10:1 à 1:1 ; et plus préférablement de 5:1 à 1:1 en poids ou l'étape de formation d'un film (étape E) est mise en œuvre par couchage à la lame, revêtement en solvant, stratification, revêtement par pulvérisation, extrusion, moulage par injection, ou une combinaison de ceux-ci.

15. Film dégradable de poly(ester-urée) à base d'acides aminés chargé de médicament selon la revendication 1 dans lequel ledit film est formé en une enveloppe, un sachet ou une poche, qui est dimensionné pour recevoir un dispositif médical implantable.
